(19) Europäisches Patentamt
European Patent Office
Office européen des brevets

(11) **EP 2 513 027 B1**

(12) **FASCICULE DE BREVET EUROPEEN**

(45) Date de publication et mention de la délivrance du brevet:
**13.08.2014 Bulletin 2014/33**

(21) Numéro de dépôt: **10805799.3**

(22) Date de dépôt: **13.12.2010**

(51) Int Cl.:
*C07C 45/52* (2006.01)     *C07C 51/25* (2006.01)
*C07C 47/22* (2006.01)     *C07C 57/04* (2006.01)

(86) Numéro de dépôt international:
**PCT/FR2010/052692**

(87) Numéro de publication internationale:
**WO 2011/080447 (07.07.2011 Gazette 2011/27)**

(54) **PROCEDE DE FABRICATION D'ACROLEINE ET/OU D'ACIDE ACRYLIQUE A PARTIR DE GLYCEROL**

VERFAHREN ZUR HERSTELLUNG VON ACROLEIN UND/ODER ACRYLSÄURE AUS GLYCEROL

PROCESS FOR MANUFACTURING ACROLEIN AND/OR ACRYLIC ACID FROM GLYCEROL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.03.2010 US 314234 P**
**14.12.2009 FR 0958937**

(43) Date de publication de la demande:
**24.10.2012 Bulletin 2012/43**

(73) Titulaire: **Arkema France**
**92700 Colombes (FR)**

(72) Inventeurs:
• **DEVAUX, Jean-François**
**F-69510 Soucieu En Jarrest (FR)**
• **FAUCONET, Michel**
**F-57730 Valmont (FR)**
• **TLILI, Nabil**
**F-57500 Saint-Avold (FR)**
• **HALLER, Philippe**
**F-57500 Saint-Avold (FR)**
• **COMBET, Jean-Paul**
**F-69400 Villefranche Sur Saone (FR)**

(74) Mandataire: **Bonnel, Claudine et al**
**ARKEMA FRANCE**
**Département Propriété Industrielle**
**420, rue d'Estienne d'Orves**
**92705 Colombes Cedex (FR)**

(56) Documents cités:
**FR-A1- 2 909 999     US-A1- 2003 199 711**
**US-A1- 2009 134 357     US-B1- 6 348 638**

EP 2 513 027 B1

**Description**

Domaine de l'invention

La présente invention concerne la fabrication d'acroléine et d'acide acrylique bioressourcés à partir de glycérol comme matière première et s'inscrit plus particulièrement dans le cadre d'un procédé de fabrication d'acroléine et d'acide acrylique suivant lequel on effectue la réaction de déshydratation de glycérol en acroléine et on met en oeuvre une étape d'oxydation d'une phase riche en eau séparée du milieu réactionnel issu de cette réaction de déshydratation, avant son recyclage à l'étape de déshydratation du glycérol. Ce traitement d'oxydation permet d'éviter l'accumulation d'impuretés organiques au cours du procédé tout en minimisant la consommation d'eau et le rejet de flux aqueux pollués.

Technique antérieure

Le procédé de synthèse de l'acide acrylique le plus largement exploité industriellement utilise une réaction catalytique du propylène à l'aide d'un mélange contenant de l'oxygène. Cette réaction est conduite généralement en phase vapeur, et le plus souvent en deux étapes : la première étape réalise l'oxydation sensiblement quantitative du propylène en un mélange riche en acroléine, dans lequel l'acide acrylique est minoritaire, puis la deuxième étape réalise l'oxydation sélective de l'acroléine en acide acrylique. Les conditions de réaction de ces deux étapes, réalisées dans deux réacteurs en série ou dans les 2 zones de réaction d'un seul réacteur « single », sont différentes et nécessitent des catalyseurs adaptés à chacune des réactions.

Depuis quelques années, les industriels conduisent des travaux de recherche et de développement sur des procédés de synthèse de l'acroléine et de l'acide acrylique utilisant des matières premières bioressourcées. A l'origine de ces travaux est le souci d'éviter d'utiliser à l'avenir les matières premières fossiles, tel que le propylène dont l'origine pétrolière contribue au réchauffement climatique dû à l'effet de serre. Par ailleurs, son coût ne peut à l'avenir qu'augmenter avec la diminution des réserves pétrolières mondiales.

Parmi ces procédés alternatifs à partir de matières premières non fossiles, on peut citer les procédés utilisant comme matière première l'acide 3-hydroxypropionique obtenu par fermentation de glucose ou de mélasse provenant de la biomasse.

On peut citer également les procédés à partir de glycérol (appelé aussi glycérine), issu de la méthanolyse des huiles végétales en même temps que les esters méthyliques qui sont, eux, employés notamment comme carburants ou combustibles dans le gazole et le fioul domestique. Ce glycérol est un produit naturel qui jouit d'une aura "verte", il est disponible en grande quantité et peut être stocké et transporté sans difficulté. La méthanolyse des huiles végétales ou des graisses animales peut s'effectuer selon différents procédés bien connus, notamment en utilisant une catalyse homogène telle que la soude ou le méthylate de sodium en solution dans le méthanol, ou en utilisant une catalyse hétérogène. On pourra se reporter sur ce sujet à l'article de D. Ballerini et al. dans l'Actualité Chimique de nov-déc 2002.

Les procédés utilisant l'acide 3-hydroxypropionique comme matière première ont un inconvénient majeur du point de vue économique. Ils mettent en jeu une réaction de fermentation qui est réalisée nécessairement en condition très diluée dans l'eau. Pour obtenir l'acide acrylique, une quantité très importante d'eau doit être éliminée par distillation, au prix d'un coût énergétique très important. Par ailleurs, l'énergie dépensée pour séparer l'eau, énergie produite à partir de matière fossile, vient dégrader fortement l'avantage initial de produire l'acide acrylique à partir de cette matière première bioressourcée. On peut citer dans ce domaine la demande WO2006/092271 qui décrit un procédé de production de polymères à partir d'acide acrylique préparé par voie enzymatique, notamment à partir de carbohydrate.

Le glycérol est reconnu aujourd'hui comme matière première adaptée pour envisager la fabrication industrielle de l'acroléine et de l'acide acrylique bioressourcés.

La réaction mise enjeu pour obtenir l'acroléine à partir du glycérol est :

$$CH_2OH\text{-}CHOH\text{-}CH_2OH \rightarrow CH_2\text{=}CH\text{-}CHO + 2\ H_2O$$

Cette étape est suivie d'une oxydation de l'acroléine pour obtenir l'acide acrylique.

Différents procédés de synthèse de l'acroléine à partir du glycérol sont décrits dans la littérature. On peut citer les documents FR 695931 ; US 2,558,520 ; WO 99/05085 ; US 5,387,720 ; WO 06/087083 ; WO 06/087084 ; WO 09/044081.

Dans la demande de brevet EP 1 710 227, le produit de réaction résultant de la réaction de déshydratation du glycérol en phase gaz, est soumis à une étape ultérieure d'oxydation en phase gaz pour obtenir de l'acide acrylique. Le procédé est mis en oeuvre dans deux réacteurs en série, comportant chacun un catalyseur adapté à la réaction mise en oeuvre. Il est préconisé d'ajouter de l'oxygène au mélange gazeux alimentant le second réacteur, afin d'améliorer la réaction d'oxydation et obtenir l'acide acrylique avec un rendement élevé. Ce procédé en deux étapes est mis en oeuvre avec du glycérol pur ou avec des solutions aqueuses comportant plus de 50 % en poids de glycérol. Il est conseillé d'utiliser une solution de glycérol concentrée afin de limiter le coût énergétique lié à l'évaporation de la solution aqueuse et le coût lié au traitement des eaux résiduaires. Cependant, si la concentration en glycérol est trop élevée, il risque de se produire davantage de réactions parasites conduisant à de nombreux sous-produits, comme la formation d'éthers de glycérol, ou des réactions entre l'acroléine ou l'acide acrylique produits et le glycérol. Ces sous-produits lourds ont tendance à rester sur le catalyseur de déshydratation et conduisent au cokage du catalyseur et à sa désactivation très rapide.

La demande WO 06/136336 décrit un procédé de synthèse d'acroléine et d'acide acrylique dans lequel la réaction de déshydratation est suivie d'une étape de séparation en une phase riche en acroléine et une phase appauvrie en acroléine, cette dernière phase, riche en eau, étant renvoyée en amont du réacteur de déshydratation afin de diluer le glycérol et obtenir une phase aqueuse comportant moins de 10% en glycérol.

Dans cette demande WO 06/136336 qui a trait essentiellement à un procédé de déshydratation en phase liquide, la phase appauvrie en acroléine et riche en eau contient aussi des composés plus lourds formés pendant la réaction de déshydratation qui ont tendance à former dans l'étape de réaction des composés lourds qui encrassent le catalyseur et provoquent sa désactivation.

La demande internationale WO 2006/092272 décrit un procédé de préparation d'acide acrylique à partir de glycérol comportant soit une étape de déshydratation du glycérol en phase liquide, soit une étape de déshydratation en phase gaz. Le mélange réactionnel contenant l'acroléine obtenue à partir de la réaction de déshydratation du glycérol est mis en contact avec de l'eau dans une unité de quench avant d'être adressé au réacteur d'oxydation. En présence d'un flux important d'eau, le catalyseur d'oxydation de l'acroléine risque de perdre rapidement son efficacité et sa tenue mécanique rendant la maintenance d'un tel procédé difficile. Selon la figure 5 de ce document, le mélange réactionnel issu de la déshydratation en phase liquide, est soumis à une distillation séparant, d'une part les produits légers de point d'ébullition inférieur à celui de l'acroléine, d'autre part une fraction comportant les produits lourds de point d'ébullition supérieur à celui de l'acroléine, cette seconde fraction, riche en eau, étant renvoyée à l'étape de réaction après avoir éliminé les impuretés dans un séparateur équipé d'une membrane. Le principe d'un tel recyclage peut néanmoins conduire à l'accumulation de certaines impuretés dans la boucle d'eau ainsi générée, en raison du manque de sélectivité de la membrane ou de son encrassement.

La demande WO 08/087315 décrit un procédé de préparation d'acide acrylique à partir de glycérol en deux étapes, dans lequel on met en oeuvre une étape intermédiaire consistant à condenser au moins en partie l'eau et les sous-produits lourds présents dans le flux issu de la première étape de déshydratation, avant d'adresser le flux au réacteur d'oxydation. Ce procédé permet l'utilisation de solutions aqueuses diluées de glycérol, produisant un effet bénéfique sur la réaction de déshydratation, tout en limitant une dégradation possible du catalyseur d'oxydation d'acroléine en présence d'une trop grande quantité d'eau. Le flux aqueux ainsi généré par l'étape de condensation est envoyé, en tout ou partie, soit vers une colonne de rectification pour récupérer les produits légers éventuellement présents, soit vers une station de traitement des eaux usées présentant cependant l'inconvénient de traitements coûteux avant rejet dans le milieu naturel de quantités importantes d'effluents aqueux. Alternativement, ce flux peut être envoyé vers un oxydeur thermique où il est incinéré. L'utilisation des évents gazeux de l'oxydation thermique n'est pas décrite. Dans un autre cas de figure, une partie de ce flux aqueux peut être recyclée directement pour diluer le glycérol à la concentration souhaitée, auquel cas, il peut y avoir accumulation d'impuretés dans la boucle d'eau ainsi formée et risque de cokage du catalyseur de déshydratation.

Dans le document US 6,348,638, une oxydation thermique est mise en oeuvre sur un flux gazeux contenant des produits secondaires de point d'ébullition inférieur à celui de l'acide acrylique, dont l'acroléine, formé au cours de la préparation d'acide acrylique à partir de propylène. Cette phase ne contient pas une proportion importante d'eau et des sous-produits lourds de point d'ébullition supérieur à celui de l'acroléine, tels que ceux issus de la déshydratation de glycérol. Par ailleurs, ce document ne décrit pas la réinjection (ou réutilisation) dans le procédé des évents gazeux de l'oxydation thermique. Une récupération d'énergie est envisagée via l'utilisation de plusieurs échangeurs de chaleur entre les évents gazeux d'une part et des flux dans le procédé d'autre part. L'utilisation de plusieurs échangeurs de chaleur rend le procédé coûteux.

La présente invention se propose de remédier aux inconvénients que présentent les procédés de fabrication d'acide acrylique sus-mentionnés, afin d'améliorer significativement le procédé de fabrication de l'acide acrylique comportant une première étape de déshydratation du glycérol en acroléine, suivie d'une étape d'oxydation de l'acroléine en acide acrylique, sur les points suivants :

- réduction de la consommation d'eau tout en assurant une optimisation de la réaction de déshydratation du glycérol en présence d'eau,
- limitation de rejets aqueux pollués,
- réduction de la consommation énergétique et de la taille des équipements,
- limitation des pertes de produit tout en assurant une récupération efficace des produits de réaction,
- augmentation de la durée de cycle des catalyseurs de déshydratation et d'oxydation.

A cet effet, il est proposé de séparer le flux aqueux issu de la réaction de déshydratation de glycérol en une phase riche en acroléine et une phase aqueuse appauvrie en acroléine et riche en eau et sous-produits lourds, et d'envoyer ladite phase aqueuse dans un oxydeur avant d'être recyclée au moins en partie à l'étape de déshydratation, permettant ainsi de limiter la consommation d'eau et l'accumulation d'impuretés organiques lourdes sur le catalyseur de déshydratation, mais également de récupérer les calories générées par l'oxydation des impuretés organiques contenues dans la phase aqueuse

Résumé de l'invention

La présente invention a donc pour objet un procédé de fabrication d'acroléine à partir de glycérol comprenant au moins les étapes suivantes :

a) on soumet du glycérol à une réaction de déshydratation pour obtenir un flux aqueux contenant de l'acroléine,
b) on sépare le flux issu de l'étape a) en une phase riche en acroléine et une phase aqueuse appauvrie en acroléine,
c) on recycle tout ou partie de la phase aqueuse appauvrie en acroléine à l'étape a), caractérisé en ce que l'on met en oeuvre une étape d'oxydation en présence d'oxygène, d'un gaz contenant de l'oxygène, de peroxyde d'hydrogène ou d'ozone, sur ladite phase aqueuse appauvrie en acroléine avant d'être recyclée à l'étape a).

L'invention a aussi pour objet un procédé de fabrication d'acide acrylique à partir de glycérol comprenant au moins les étapes suivantes :

a) on soumet du glycérol à une réaction de déshydratation pour obtenir un flux aqueux contenant de l'acroléine,
b) on sépare le flux issu de l'étape a) en une phase riche en acroléine et une phase aqueuse appauvrie en acroléine,
c) on recycle tout ou partie de la phase aqueuse appauvrie en acroléine à l'étape a),
d) on soumet la phase riche en acroléine à une réaction d'oxydation catalytique pour obtenir un flux contenant de l'acide acrylique,
e) on soumet le flux issu de l'étape d) à un ou plusieurs traitements de purification, et l'on récupère l'acide acrylique purifié,

caractérisé en ce que l'on met en oeuvre une étape d'oxydation en présence d'oxygène, d'un gaz contenant de l'oxygène, de peroxyde d'hydrogène ou d'ozone, sur ladite phase aqueuse appauvrie en acroléine avant d'être recyclée à l'étape a).
D'autres caractéristiques et avantages de l'invention ressortiront mieux à la lecture de la description détaillée qui suit et des exemples de réalisation non limitatifs de l'invention, en référence aux figures annexées qui représentent :

Figure 1 : schéma de principe du procédé de fabrication d'acroléine selon l'invention.
Figure 2 : schéma de principe du procédé de fabrication d'acide acrylique selon l'invention.
Figure 3 : schéma détaillé d'un mode de réalisation préféré du procédé de fabrication d'acroléine selon l'invention.
Figure 4 : schéma détaillé des étapes d) et e) du procédé de fabrication d'acide acrylique selon l'invention.
Figure 5 : schéma détaillé d'un mode de réalisation préféré du procédé de fabrication d'acide acrylique selon l'invention.

Description détaillée de l'invention

Fabrication de l'acroléine

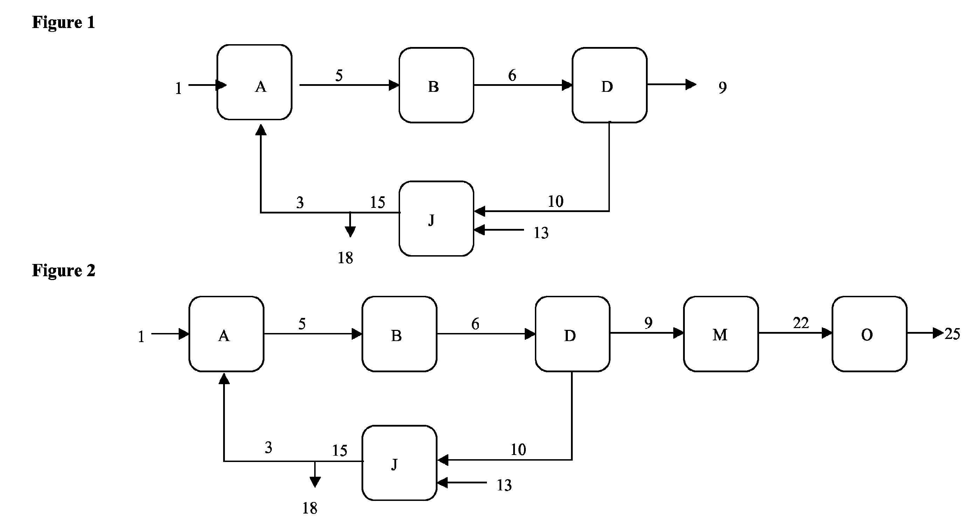

En référence à la Figure 1, pour la mise en oeuvre du procédé selon l'invention, on utilise généralement pour alimenter le réacteur (B) de l'étape a) de déshydratation de glycérol, un flux (5) contenant le glycérol et de l'eau, avec un ratio massique eau/glycérol pouvant varier dans de larges mesures, par exemple entre 0,04 / 1 et 9 / 1, et de préférence entre 0,7 / 1 et 5 / 1. Le flux (5) peut également contenir de l'oxygène, de l'azote et du $CO_2$. Ce flux (5) est avantageusement obtenu lors d'une étape de mélange (A) d'un flux (1) riche en glycérol et d'une phase (3) riche en eau recyclée et pouvant contenir de l'azote, oxygène, argon et du $CO_2$. Le flux (1) peut être par exemple du glycérol brut commercial (glycérine), c'est-à-dire contenant typiquement 80-90% de glycérol, 1 à 10% de sels, 1 à 4% de matières organiques non glycérineuses dont le méthanol, et 3 à 15% d'eau. Avantageusement, on utilise du glycérol dessalé, qui peut être obtenu à partir de glycérol brut par tout moyen connu de l'homme de l'art, comme une distillation sous pression réduite ou un flash sous pression réduite ou une séparation utilisant des résines échangeuses d'ion tel que décrit par exemple dans la demande EP1978009. On peut aussi partir de glycérine sans sel obtenue par des procédés de transestérification d'huiles catalysés par des catalyseurs hétérogènes. On peut aussi utiliser de la glycérine raffinée d'une pureté supérieure à 98%, 99% ou 99,5%. On peut aussi utiliser une solution aqueuse contenant de 20% à 99%, de préférence de 30% à 80% en poids de glycérol.

La réaction de déshydratation, étape a), qui est favorisée par un niveau de température élevée, est effectuée en général en phase gaz dans le réacteur (B) en présence d'un catalyseur à une température allant de 150°C à 500°C, de préférence comprise entre 250°C et 350°C et une pression comprise entre $10^5$ et $5.10^5$ Pa (1 et 5 bars). Elle peut également être conduite en phase liquide, dans ce cas la température est comprise entre 150°C et 350°C sous une pression allant de $5.10^5$ à $100.10^5$ Pa. De préférence, on effectue cette première étape en phase gaz.

On peut aussi l'effectuer en présence d'oxygène ou d'un gaz contenant de l'oxygène comme décrit dans les demandes WO 06/087083 et WO 06/114506. Dans ce cas, la quantité d'oxygène est choisie de façon à être en dehors du domaine d'inflammabilité en tout point de l'installation. Le rapport molaire entre l'oxygène moléculaire et le glycérol est généralement de l'ordre de 0,1 à 1,5, de préférence de 0,3 à 1,0. L'oxygène ou le gaz contenant de l'oxygène peut être appporté en partie ou en totalité par le flux (3).

La réaction de déshydratation peut aussi être mise en oeuvre dans un milieu réactionnel comportant une phase gazeuse contenant de 1 et 3 000 ppm d'un composé acide au sens de la classification de Pearson choisi par exemple parmi $SO_3$, $SO_2$, $NO_2$, la réaction de déshydratation étant conduite soit en phase gaz soit en phase liquide.

La réaction de déshydratation du glycérol est généralement effectuée sur des catalyseurs solides acides. Les catalyseurs qui conviennent sont des matériaux homogènes ou multiphases, insolubles dans le milieu réactionnel qui ont une acidité de Hammett, notée $H_0$ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse.

Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides.

Ces catalyseurs pourront en particulier être constitués par un sel d'hétéropolyacide dans lequel des protons dudit hétéropolyacide sont échangés avec au moins un cation choisi parmi les éléments appartenant aux Groupes 1 à XVI de la Classification Périodique des Eléments, ces sels d'hétéropolyacide contenant au moins un élément choisi parmi le groupe comprenant W, Mo et V.

Parmi les oxydes mixtes, on peut citer particulièrement ceux à base de fer et de phosphore et ceux à base de césium, phosphore et tungstène.

Les catalyseurs sont notamment choisis parmi les zéolithes, les composites Nafion® (à base d'acide sulfonique de polymères fluorés), les alumines chlorées, les acides et sels d'acides phosphotungstiques et/ou silicotungstiques, et différents solides de type oxydes métalliques tels que oxyde de tantale $Ta_2O_5$, oxyde de niobium $Nb_2O_5$, alumine $Al_2O_3$, oxyde de titane $TiO_2$, zircone $ZrO_2$, oxyde d'étain $SnO_2$, silice $SiO_2$ ou silico-aluminate $SiO_2$-$Al_2O_3$, imprégnés de fonctions acides telles que borate $BO_3$, sulfate $SO_4$, tungstate $WO_3$, phosphate $PO_4$, silicate $SiO_2$, ou molybdate $MoO_3$' ou un mélange de ces composés.

Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni, or montmorillonite.

Les catalyseurs préférés sont les zircones phosphatées, les zircones tungstées, les zircones silicées, les oxydes de titane ou d'étain imprégnés de tungstate ou phosphotungstate ou silicotungstate, les alumines ou silices phosphatées, les hétéropolyacides ou sels d'hétéropolyacides, les phosphates de fer et les phosphates de fer comprenant un promoteur.

On peut aussi effectuer la réaction de déshydratation du glycérol en présence d'une quantité d'hydrogène allant de 0,1 à 10% en volume par rapport au mélange réactionnel, et dans ce cas en présence d'un catalyseur choisi parmi ceux décrit dans la demande US 2008/018319.

Le réacteur (B) utilisé peut fonctionner en lit fixe, en lit mobile, en lit fluidisé ou en lit fluidisé circulant, ou dans une configuration en modules (plaques ou paniers). La durée de contact exprimée en secondes est le rapport entre le volume du lit de catalyseur et le volume des réactifs gazeux envoyés par seconde. Les conditions moyennes de température et de pression existant dans un lit peuvent varier selon la nature du catalyseur, la nature du lit catalytique et la dimension du catalyseur. En général, la durée de contact est de 0,1 à 20 secondes et de préférence de 0,3 à 15 secondes.

A l'issue de l'étape a), on obtient un flux aqueux (6), pouvant être liquide ou gazeux, contenant l'acroléine recherchée, de l'eau, du glycérol n'ayant pas réagi, et des sous-produits tels que hydroxypropanone, propanaldéhyde, acétaldéhyde, formaldéhyde, acide acrylique, acide propionique, acide acétique, acide formique, acétone, phénol, des produits d'addition de l'acroléine sur le glycérol, des produits de polycondensation du glycérol, des éthers de glycérol cycliques, ainsi que des composés légers tels que azote, oxygène, monoxyde et dioxyde de carbone, argon. Certains de ces produits sont des composés lourds, d'autres sont des composés légers condensables. Pour d'autres, il s'agit de composés légers incondensables dans les conditions de températures et de pression habituellement mises en oeuvre.

Le flux (6) a une teneur en eau importante due à la composition du flux (5) entrant dans le réacteur (charge de glycérol) et à la réaction elle-même (déshydratation). L'étape b) dans le procédé selon l'invention consiste à séparer ce flux (6) en une phase (9) enrichie en acroléine et une phase (10) riche en eau et appauvrie en acroléine. Cette étape b), telle que la condensation partielle de l'eau décrite par exemple dans la demande de brevet WO 08/087315 au nom de la Société Déposante, ou telle que la séparation décrite dans la demande WO 2006/136336, a pour but d'éliminer la majeure partie de l'eau présente et les sous-produits lourds avant d'envoyer le flux contenant l'acroléine vers une étape de purification dans un procédé de fabrication d'acroléine ou à l'étape d'oxydation de l'acroléine en acide acrylique dans un procédé de fabrication d'acide acrylique à partir de glycérol en deux étapes. Cette séparation partielle de l'eau permet ainsi d'éviter une dégradation du catalyseur de 2ème étape d'oxydation de l'acroléine en acide acrylique, et d'éviter lors des étapes ultérieures l'élimination de grandes quantités d'eau, qui risque d'être coûteuse et d'entrainer des pertes en acide acrylique. En outre, elle permet d'éliminer une partie des impuretés « lourdes » formées lors de la déshydratation du glycérol.

Cette étape b) est réalisée sur une unité de séparation (D). Dans le cas où l'étape a) a été réalisée en phase gaz, l'unité de séparation (D) est une installation de condensation qui peut être une colonne d'absorption couplée ou non à un évaporateur, un échangeur de chaleur, un condenseur, un déflegmateur, ainsi que tout appareillage bien connu de l'homme de l'art permettant de réaliser une condensation partielle d'un flux aqueux. Elle est conduite dans des conditions telles que 20% à 95%, de préférence 40% à 90% de l'eau présente dans le flux (6) est éliminée dans le flux (10) liquide. Le flux gazeux (9) contient généralement plus de 80% et de préférence plus de 90% de l'acroléine initialement contenue dans le flux (6). Ce résultat est obtenu en abaissant la température à une température de 60 à 120°C.

Dans le cas où l'étape a) a été réalisée en phase liquide sous pression, l'étape b) peut être réalisée par une détente à une pression de 1 à 4 bars éventuellement couplée à un échangeur de chaleur et une installation de séparation gaz liquide qui peut être un ballon de flash, une colonne à distiller ou tout autre dispositif connu de l'homme de l'art. On récupère un flux liquide (10) qui contient 20% à 95%, de préférence 40% à 90% de l'eau présente dans le flux (6) et un flux gazeux (9) qui contient plus de 80% et de préférence plus de 90% de l'acroléine initialement contenue dans le flux (6).

La phase condensée (10) ainsi générée contient généralement de 90% à 99% d'eau, le reste représentant de l'acroléine et des impuretés telles que acide acrylique, glycérol, acide acétique, hydroxypropanone, acide propionique, et autres composés organiques lourds.

Un des objets du procédé de l'invention est d'obtenir une phase aqueuse riche en eau et appauvrie en acroléine (10) qui soit recyclable au moins en partie à l'étape de réaction sous forme de flux (3), exempte d'impuretés lourdes néfastes pour le catalyseur de déshydratation.

Selon le procédé de l'invention, la phase aqueuse (10) est soumise à une étape d'oxydation (J) en présence d'oxygène, d'un gaz contenant de l'oxygène, de peroxyde d'hydrogène ou d'ozone, de préférence en présence d'oxygène ou d'un gaz contenant de l'oxygène, conduisant à un flux (15) comprenant essentiellement de l'eau, de l'oxygène et du $CO_2$ résultant de la dégradation ultime des composés organiques et éventuellement de l'azote et de l'argon. Ce flux (15), tout ou en partie, peut alors être avantageusement recyclé à l'étape a) de déshydratation du glycérol, sans risque d'accumulation d'impuretés sur le catalyseur de déshydratation ; il permet notamment d'ajuster la teneur en eau du flux (5) contenant le glycérol qui va alimenter le réacteur de déshydratation (B). De préférence une partie (18) du flux (15) est éliminée du procédé. Le flux (18) comprend notamment une partie de l'eau générée par les réactions de déshydratation et d'oxydation des matières organiques et le $CO_2$ générée par l'oxydation des matières organiques Dans la mesure où ce flux ne contient pas de polluant, il peut être rejeté dans le milieu naturel. Une partie de l'énergie contenue dans le flux de sortie de l'oxydeur (J) sous la forme de flux (15) peut servir à préchauffer l'alimentation (10) et/ou (13) de ce même oxydeur (J). De plus, une partie de l'énergie contenue dans le flux de sortie de l'oxydeur (J) sous forme de flux (3) recyclé à l'étape de déshydratation a) peut servir à préchauffer la solution de glycérol dans l'étape de mélange (A) avant d'envoyer le flux réactif dans le réacteur de déshydratation (B). Dans le cas où l'étape de déshydratation a) est effectuée en phase gaz, l'énergie contenue dans le flux de sortie de l'oxydeur (J) sous forme de flux (3) peut servir à vaporiser la solution de glycérol dans l'étape de mélange (A) avant d'envoyer le flux réactif dans le réacteur de déshydratation (B). On notera que dans ce cas le transfert d'énergie du flux (3) au glycérol (1) se fait lors du mélange des deux flux et ne nécessite donc pas d'échangeur de chaleur.

L'étape d'oxydation (J) consistant à transformer les impuretés présentes dans le flux (10) à l'état de $CO_2$ et $H_2O$ peut être conduite de différentes manières :

Selon un premier mode de réalisation de l'invention, on conduit une oxydation thermique en phase gaz en présence d'oxygène à une température supérieure à 700°C, dans un système d'oxydation thermique (oxydeur), constitué habituellement d'une chambre de combustion munie d'un brûleur et d'un échangeur primaire permettant de préchauffer l'effluent à traiter en utilisant l'énergie contenue dans les fumées sortant de la chambre de combustion. Le flux est introduit dans la chambre de combustion où la température est maintenue à une température supérieure à la température d'autoinflammation des impuretés à oxyder, généralement à plus de 700°C, de préférence à plus de 750°C avec appoint éventuel de combustible tel que du gaz naturel, propane ou du fioul léger, moyen ou lourd, de façon à entretenir la combustion si la concentration en composés organiques n'est pas suffisante. L'oxygène nécessaire à la réaction est apporté par de l'oxygène pur, de l'air enrichi en oxygène ou de l'air. L'oxygène et le flux à traiter devant constituer un mélange le plus homogène possible, il est préférable d'ajouter l'oxygène en amont du réacteur d'oxydation. Le temps de séjour des gaz à la température requise est typiquement de l'ordre de 0,6 à 2 secondes.

Selon un second mode de réalisation de l'invention, on conduit une oxydation en phase gaz en présence d'oxygène à une température allant de 200°C à 500°C en présence d'un catalyseur. Comme catalyseurs d'oxydation, on utilisera typiquement des catalyseurs solides constitués d'une espèce active déposée sur un support inorganique, par exemple de l'alumine ou de la silice ou un support métallo-céramique. Les espèces actives sont à base de métaux précieux (platine, palladium ou rhodium, ou une combinaison de ces métaux) ou bien d'oxydes métalliques à base de chrome, fer, molybdène, tungstène, manganèse, cobalt, cuivre ou nickel pouvant être dopés avec des métaux précieux. Les catalyseurs peuvent se présenter sous forme de billes, pastilles, granulés, extrudés, briques ou monolithes. On respectera typiquement des vitesses spatiales de l'ordre de 10000 à 50000 h$^{-1}$ (rapport débit de gaz / volume de catalyseur). Selon ce mode d'oxydation, il est possible également d'ajouter un combustible tel que du gaz naturel ou du fioul léger, moyen ou lourd, de façon à entretenir la combustion si la concentration en composés organiques n'est pas suffisante.

Selon un troisième mode de réalisation de l'invention, on conduit une oxydation en voie humide (oxydation sous-critique) ou une oxydation supercritique, à une température supérieure à 150°C et une pression supérieure à 5 bars. L'oxydation en voie humide est réalisée à des températures comprises entre 150°C et 330°C et des pressions de 5 à 150 bars. Le temps de séjour dans le réacteur est typiquement de 30 minutes à 3 heures. Avantageusement on utilisera un catalyseur hétérogène à base d'oxydes métalliques ou d'oxydes mixtes à surface spécifique de 10 à 1000 m$^2$/g contenant un composé actif dispersé tel Pt, Pd, Rh, Ru, Cu, Mn, Co. La vitesse spatiale (débit à traiter divisé par volume de catalyseur) est typiquement comprise entre 0,5 et 10 h$^{-1}$. De l'oxygène, de l'air enrichi en oxygène ou de l'air est injecté dans le réacteur ou en amont du réacteur. L'oxydation supercritique, quant à elle, est réalisée à une température supérieure à 374 °C et une pression supérieure à 221 bars.

Selon un quatrième mode de réalisation de l'invention, on conduit l'oxydation en phase liquide en présence de peroxyde d'hydrogène ou d'ozone, ou la combinaison de ces deux réactifs. Ces réactifs peuvent être activés par exemple par l'utilisation de rayonnement UV ou par l'utilisation de catalyseurs tels les sels de fer II.

Dans tous les modes de réalisation de l'invention, on obtient un flux (3) qui peut être avantageusement recyclé totalement ou en partie à l'étape a) de déshydratation du glycérol, sans risque d'accumulation d'impuretés sur le catalyseur de déshydratation ; ce flux (3) permet notamment d'ajuster la teneur en eau du flux (5) contenant le glycérol qui va alimenter le réacteur de déshydratation (B).

Dans tous les cas, l'énergie contenue dans le flux de sortie de l'oxydeur est avantageusement utilisée au moins en partie pour préchauffer le flux d'entrée de l'oxydeur. Elle peut aussi être utilisée pour préchauffer les flux de matière qui entrent dans la première étape de réaction, tel que le glycérol, l'eau et les gaz inertes.

Dans le cas d'une réaction en phase gaz et d'un oxydeur en phase gaz, on utilise avantageusement les calories à un niveau élevé du flux (3) pour vaporiser le flux de glycérol (1), qui bout à une température très élevée. Par ailleurs l'eau nécessaire à la réaction dans le flux (5) est apportée directement à l'état gazeux à un niveau thermique élevé, ce qui est très avantageux en terme énergétique.

Selon un mode préféré, l'oxydeur thermique ou catalytique en phase gaz (J) sera opéré à une pression supérieure à celle du réacteur (B) de 0,1 à 5 bars et de préférence de 0,2 à 2 bars. Selon un autre mode préféré, l'oxydeur thermique ou catalytique sera opéré à une pression voisine de la pression atmosphérique et le flux gazeux (3) est comprimé avant d'être injecté dans le réacteur (B).

Selon un mode de réalisation du procédé selon l'invention (non représenté sur la figure 1), la phase (9) enrichie en acroléine, qui est débarrassée des sous-produits lourds et de l'essentiel de l'eau, provenant de l'étape b) de séparation du flux issu de l'étape de déshydratation a), est soumise à un traitement de purification comprenant des étapes d'absorption/distillation, telles que celles décrites par exemple pour le flux d'acroléine produit par oxydation du propylène dans le document Techniques de l'Ingénieur, Traité des Procédés, J 6 100 1-4.

La purification du flux (9) contenant l'acroléine, après refroidissement par un ou plusieurs échangeurs de chaleur, comprend en général une absorption dans de l'eau ou un flux aqueux recyclé pour laisser partir en tête les incondensables et récupérer en pied une solution aqueuse d'acroléine diluée.

Cette absorption peut être réalisée dans une colonne à garnissage ou à plateau, de préférence à contre-courant. Avantageusement, on élimine en tête de la colonne les composés légers incondensables, tels que azote, oxygène, monoxyde et dioxyde de carbone.

La solution aqueuse d'acroléine est ensuite séparée par distillation. Pour cela, on peut utiliser un enchaînement de colonnes à distiller, comme décrit par exemple dans le brevet US 3,433,840 ou une seule colonne comme décrit par exemple dans les documents EP1300384 ou EP1474374. Cette distillation permet de récupérer, d'une part un flux constitué majoritairement d'eau dont la majeure partie est généralement recyclée à l'étape d'absorption, et d'autre part un flux gazeux ou liquide contenant une teneur massique en acroléine supérieure à 80% et de préférence > 94% et une teneur massique en eau inférieure à 15% par rapport à l'acroléine et de préférence < 5%.

La purification du flux (9) contenant l'acroléine peut aussi être réalisée simplement par distillation sans absorption préalable dans l'eau. Cette alternative est avantageusement mise en oeuvre lorsque le flux (9) contient peu de gaz incondensables.

Le flux d'acroléine, liquide ou gazeux, obtenu à l'issu des étapes de purification du flux (9), peut alors être utilisé pour préparer du méthylmercaptopropionaldéhyde (MMP) par réaction avec du méthyl mercaptan en présence d'un catalyseur. La réaction du MMP, éventuellement purifié, avec de l'acide cyanhydrique ou du cyanure de sodium effectuée selon les synthèses de Bücherer ou de Strecker bien connues de l'homme du métier, conduit alors, soit à la méthionine, soit à l'hydroxyanalogue de la méthionine, après transformation du produit de réaction, comme décrit dans le document Techniques de l'Ingénieur, Traité Génie des procédés, J 6 410-1 à 9.

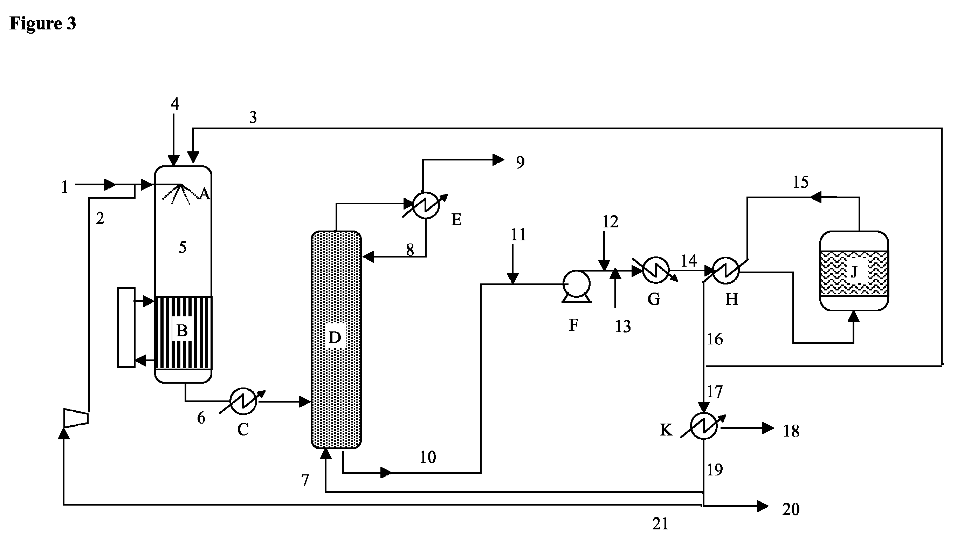

La figure 3 représente un schéma détaillé d'un mode de réalisation préféré du procédé de fabrication d'acroléine selon l'invention, la réaction de déshydratation du glycérol étant réalisée en phase gaz.

Le flux gazeux (5) alimentant le réacteur de déshydratation (B) est obtenu dans la chambre de mélange (A) dans laquelle s'effectue la vaporisation du glycérol à l'aide de gaz chauds issus du recyclage (3) de la phase aqueuse après le traitement d'oxydation effectué dans l'oxydeur (J), ainsi qu'éventuellement du recyclage (2) d'un flux gazeux contenant majoritairement du $CO_2$ issu de l'oxydeur (J). Le flux (1) de glycérol sous forme liquide, éventuellement préchauffé à une température de l'ordre de 100°C à 200°C pouvant aller jusqu'à 280°C, peut être injecté dans cette chambre dans une veine gazeuse à co-courant ou à contre-courant via des buses de pulvérisation ou d'atomisation qui permettent de former de fines gouttelettes au contact du flux (3) recyclé comprenant essentiellement de l'eau, de l'oxygène et du $CO_2$ et éventuellement de l'azote, ce flux étant sous forme gazeuse après le traitement d'oxydation thermique en phase gaz, ou après une phase d'évaporation dans le cas d'une oxydation thermique en phase liquide (non représenté). Les buses de pulvérisation permettent de disperser le flux de glycérol liquide sous forme de fines gouttelettes en jouant sur des effets mécaniques (taille et forme d'orifice de la buse, débit, pression). Les buses d'atomisation comprennent en plus l'injection d'un gaz dans la buse, comme par exemple le flux (2), et permettent généralement d'atteindre des tailles de gouttelettes inférieures à celles obtenues avec des buses de pulvérisation. Ces systèmes permettent de former des gouttelettes de taille inférieure au mm, et de préférence inférieure à 300 $\mu$m. Plus la taille des gouttelettes est fine, plus l'évaporation du flux (1) est rapide.

Dans un autre mode de réalisation, le mélange intime entre le flux liquide de glycérol (1) et le flux (3) recyclé est réalisé dans un mélangeur à effet venturi avant d'être injecté dans le réacteur (B).

Dans le schéma de la figure (3) l'énergie nécessaire à préchauffer le flux (5) entrant dans le réacteur est apportée par les flux (3) et (2) surchauffés. Alternativement un échangeur de chaleur pourrait être placé entre l'unité de mélange (A) et le réacteur (B).

Un apport d'oxygène, d'air ou d'un gaz contenant de l'oxygène favorisant la réaction de déshydratation est effectué en (4).

Le flux réactionnel gazeux (6) à la sortie du réacteur peut être refroidi dans un échangeur (C) à une température comprise entre 70°C et 200°C et de préférence entre 110°C et 180°C avant d'entrer dans une colonne de condensation (D), équipée d'un condenseur (E) qui permet de séparer une phase liquide (8) contenant majoritairement de l'eau et de l'acroléine qui est recyclée dans la colonne (D), et le flux gazeux (9) contenant l'acroléine produite. Généralement ce flux (9) contient de l'eau dans un ratio massique acroléine / eau allant de 1/0,02 à 1/3 et de préférence 1/0,5 à 1/2, mais aussi les sous-produits légers, tels que acétaldéhyde, propanaldéhyde, acétone et éventuellement $O_2$ et des gaz inertes CO et $CO_2$. De façon à épuiser en acroléine le flux liquide (10) sortant en pied de la colonne (D), celle-ci peut être alimentée en pied par un flux gazeux (7) contenant majoritairement du $CO_2$ issu de l'oxydeur (J) et dont le rôle est de stripper l'acroléine. D'autres flux gazeux pourraient convenir tel que de l'azote, de la vapeur ou un autre flux recyclé de l'installation. Alternativement l'épuisement de l'acroléine peut être réalisé par rebouillage en pied de la colonne (D) ou par l'adjonction d'une colonne de stripping alimentée en tête par le flux liquide (10) et en pied par un flux gazeux comme décrit précédemment.

Le flux liquide (10) sortant en pied de la colonne d'absorption, auquel on a ajouté éventuellement un flux aqueux (11) pouvant contenir du glycérol et du méthanol provenant de la purification de la matière première glycérine, est vaporisé dans les échangeurs (G) et (H) puis envoyé dans l'oxydeur (J) sous forme de gaz. Alternativement seule une partie de ce flux aqueux est vaporisée,-la partie comportant la majorité de l'eau et des légers-, puis envoyée sous forme gazeuse dans l'oxydeur (J). Le résidu liquide de vaporisation, concentré en organiques plus lourds que l'eau est, soit injecté sous forme liquide dans l'oxydeur (J), soit éliminé du procédé.

La température d'entrée de l'oxydeur (J) sera choisie pour permettre une bonne combustion des matières organiques, généralement de 200°C à 500°C dans le cas d'une oxydation catalytique en phase gaz et 600°C à 1200°C dans le cas d'une oxydation thermique. Un apport d'oxygène ou d'air ou d'air enrichi en oxygène nécessaire à l'oxydation thermique est réalisé en (13). Dans la boucle, il est possible d'incorporer un flux gazeux (12) contenant des impuretés organiques permettant ainsi d'incinérer des effluents gazeux issus par exemple de l'unité de purification de l'acide acrylique en aval du procédé de fabrication d'acroléine. L'échangeur (H) permet de récupérer l'énergie provenant des fumées (15) en sortie de l'oxydeur pour préchauffer le flux (14) à l'entrée de l'oxydeur. En sortie de l'oxydeur, le flux gazeux (16) est au moins partiellement recyclé à l'étape de déshydratation (flux (3)), le flux restant (17) étant purgé d'une partie de l'eau liquide (18) via un échangeur (K) et recyclé soit sous forme de flux gazeux (7) en pied de la colonne d'absorption (D), soit sous forme de flux gazeux (2) à l'entrée de la phase de mélange (A), comme décrit précédemment, une purge de gaz pouvant être effectuée en (20).

Fabrication de l'acide acrylique

En référence à la figure 2, pour la mise en oeuvre du procédé selon l'invention, la phase (9) enrichie en acroléine et débarrassée des sous-produits lourds et de l'essentiel de l'eau, provenant de l'étape b) de séparation du flux issu de l'étape de déshydratation a), est soumise à une réaction d'oxydation catalytique d) dans un réacteur (M) pour obtenir un flux (22) contenant l'acide acrylique recherché. Ce flux est ensuite soumis dans une étape e) à un ou plusieurs traitements de purification (O) permettant de récupérer de l'acide acrylique purifié (25).

La réaction d'oxydation de l'acroléine en acide acrylique s'effectue en présence d'oxygène moléculaire ou d'un mélange contenant de l'oxygène moléculaire, à une température allant de 200°C à 350°C, de préférence de 250°C à 320°C, et sous une pression allant de 1 à 5 bars en présence d'un catalyseur d'oxydation. Comme catalyseur d'oxydation, on utilise tous types de catalyseurs bien connus de l'homme de l'art pour cette réaction. Généralement sont utilisés des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. En particulier, sont utilisées les formulations contenant Mo et/ou V et/ou W et/ou Cu et/ou Sb et/ou Fe comme constituants principaux.

Le réacteur d'oxydation (M) peut fonctionner en lit fixe, en lit fluidisé ou en lit fluidisé circulant. Il est possible aussi d'utiliser un échangeur à plaques avec un agencement modulaire du catalyseur tel que décrit dans les documents EP 995491, EP 1147807 ou US 2005/0020851.

Le mélange gazeux (22) issu de la réaction d'oxydation est constitué, en dehors de l'acide acrylique de différents composés tels que :

- des composés légers incondensables dans les conditions de températures et de pression habituellement mises en oeuvre : $N_2$, $O_2$ non converti, CO et $CO_2$ formés en faible quantité par oxydation ultime ou tournant en rond, par recyclage, dans le procédé,
- de composés légers condensables : en particulier l'eau résiduelle de l'étape précédente, générée par la réaction de déshydratation ou présente comme diluant, l'acroléine non convertie, des aldéhydes légers, comme le formaldéhyde et l'acétaldéhyde, l'acide formique et l'acide acétique et l'acide propionique.
- des composés lourds résiduels de l'étape précédente: furfuraldéhyde, benzaldéhyde, acide et anhydride maléique, acide benzoïque, phénol, protoanémonine.

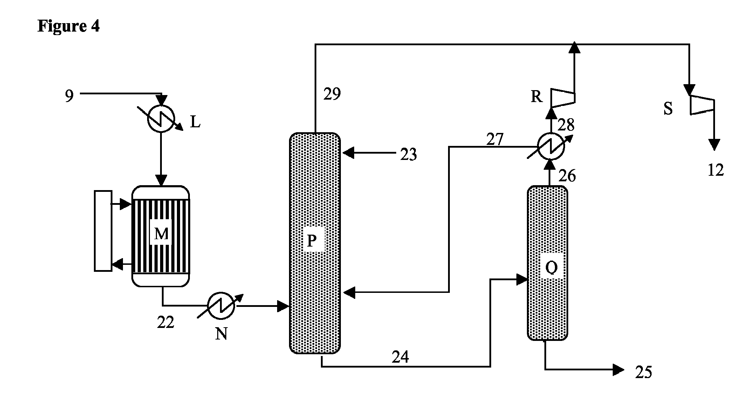

Pour obtenir de l'acide acrylique répondant à une certaine qualité technique, il est nécessaire de soumettre ce mélange (22) à un train de purification, représenté en partie par exemple sur la figure 4 :

La première étape de cette phase de purification consiste en une extraction de l'acide acrylique par absorption à contre-courant. Pour cela, on introduit le mélange gazeux (22), éventuellement après refroidissement dans un échangeur (N) en pied d'une colonne d'absorption (P) où il rencontre à contre-courant un solvant (23) introduit en tête de colonne, généralement de l'eau. Les composés légers incondensables dans les conditions de température et de pression habituellement mises en oeuvre (respectivement plus de 50°C et moins de $2.10^5$ Pa) sont éliminés en tête de cette colonne d'absorption dans un flux (29). Le solvant (23) mis en oeuvre dans cette colonne est l'eau. L'eau pourrait être remplacée par un solvant hydrophobe à haut point d'ébullition, comme il est décrit par exemple dans les brevets de FR 2.146.386 ou US 5.426.221, ainsi que dans le brevet FR 96.14397. L'eau utilisée comme solvant absorbant peut être apportée par une source extérieure au procédé, mais peut être constituée pour partie ou totalement par de l'eau issue du recyclage d'une phase aqueuse dans le procédé, par exemple l'eau séparée dans l'unité de séparation (D), ou l'eau récupérée à partir du flux de tête d'une colonne de séchage azéotropique éventuellement présente dans le train de purification. Selon une variante, il n'est pas ajouté d'eau dans la colonne d'absorption. Les conditions opérationnelles de cette étape d'absorption sont les suivantes :

Le mélange réactionnel gazeux est introduit en pied de colonne à une température comprise entre 130°C et 250°C. L'eau est introduite en tête de colonne à une température comprise entre 10°C et 60°C. Les quantités respectives d'eau et de mélange réactionnel gazeux sont telles que le ratio massique eau/ acide acrylique est compris entre 1/1et 1/4. L'opération est conduite à la pression atmosphérique.

La colonne d'absorption (P) peut être couplée avec une colonne de distillation des composés très légers, essentiellement l'acroléine non convertie à l'issue de la réaction, présente en faible concentration dans la solution aqueuse d'acide acrylique récupérée en pied de colonne d'absorption. Cette colonne de distillation (Q), fonctionnant sous une pression de $6.10^3$ à $7.10^4$ Pa, est alimentée en tête par le flux (24) de pied de colonne d'absorption précédente, et permet d'éliminer en tête un flux (26) d'acide acrylique enrichi en acroléine, qui est recyclé au moins partiellement, via un condenseur, sous forme d'un flux liquide (27) en partie inférieure de la colonne d'absorption, pour une élimination finale en tête de cette même colonne, le flux gazeux restant (28) étant recomprimé dans un compresseur (S) et envoyé éventuellement dans l'oxydeur présent dans le procédé de fabrication d'acroléine. On obtient ainsi à l'issue de ces étapes de purification un mélange aqueux (25) d'acide acrylique dans l'eau (ratio massique 1/1 à 4/1) débarrassé de l'essentiel de l'acroléine non convertie, que l'on nomme "acide acrylique brut".

Selon la qualité recherchée pour l'acide acrylique, ce mélange sera soumis à des traitements complémentaires, décrits dans de nombreux brevets, notamment à une étape de déshydratation qui est réalisée en présence d'un solvant de l'acide acrylique non miscible à l'eau. Cette étape de déshydratation peut être réalisée par distillation azéotropique du mélange solvant, eau, acide acrylique qui permet de sortir en tête de distillation l'azéotrope solvant / eau. L'acide acrylique récupéré en pied subit ensuite une distillation des composés légers (étêtage) et séparation des composés lourds (équeutage). On obtient alors une qualité d'acide acrylique dénommée « technique », qui peut ensuite subir une purification ultérieure, par exemple par cristallisation fractionnée pour donner un grade glacial.

Optimisation énergétique du procédé selon l'invention

Dans le procédé selon l'invention, sont présents des flux gazeux qui nécessitent d'être refroidis et condensés, des flux liquides qui nécessitent d'être vaporisés. L'utilisation de systèmes à compression, notamment les pompes à chaleur, permet de minimiser les calories perdues en permettant de transférer de la chaleur du milieu le plus froid vers le milieu le plus chaud. Une pompe à chaleur est un dispositif thermodynamique dont le fonctionnement est basé sur le principe de cycle à compression de fluides frigorigènes. Lorsque le fluide est comprimé et passe de l'état gazeux à l'état liquide, il se produit un phénomène exothermique (condensation) qui produit de la chaleur. A l'inverse, si on détend le fluide en le faisant passer de l'état liquide à l'état gazeux, il se produit un phénomène endothermique (évaporation) qui permet d'absorber de la chaleur et de refroidir. Tout repose sur le changement d'état utilisé en circuit fermé.

Dans le procédé de l'invention, on utilise avantageusement une pompe à chaleur pour récupérer l'énergie de condensation de l'eau du flux réactionnel (6) à la sortie du réacteur de déshydratation et pour vaporiser la phase aqueuse appauvrie en acroléine séparée de la phase enrichie en acroléine lors de l'étape b). La pompe à chaleur pourra fonctionner avec de l'eau ou avec tout fluide frigorigène adapté connu de l'homme de l'art, tel que par exemple le 1,1,1,3,3-pentafluoropropane ou le 1,1,1,3,3-pentafluoropentane, ou une composition comprenant au moins une hydrochlorooléfine tel que le 1-chloro,3,3,3-trifluoropropène ou le 2-chloro,3,3,3-trifluoropropène, ou une composition comprenant en poids de 1 à 50% de méthyltétrahydrofuranne et de 5 à 99% de nonafluorobutyl alkyl éther de formule $C_4F_9OR$, R comportant de 1 à 4 atomes de carbone, telle que décrite dans la demande de brevet FR 2 928 648.

Dans le cas où la réaction de déshydratation est réalisée en phase gaz, le flux (6) sort du réacteur sous forme d'un mélange gazeux à une température allant de 150°C à 550°C et de préférence entre 250°C et 400°C. Ce flux est refroidi grâce à un premier échangeur de chaleur pour l'amener à une température allant de 150°C à 200°C. Généralement, cet échangeur permet de récupérer de l'énergie en produisant de la vapeur basse pression. Un deuxième échangeur permet de refroidir ce flux à une température allant de 70°C à 120°C et de préférence de 90°C à 110°C afin d'alimenter la colonne d'absorption (D), d'où il sort en pied la phase appauvrie en acroléine (10), liquide, séparée de la phase gazeuse enrichie en acroléine (9). Ce flux liquide (10) est vaporisé à une pression supérieure de 0,1 à 3 bars à la pression d'entrée du réacteur de déshydratation grâce à un troisième échangeur de chaleur à une température allant de 110°C à 200°C et de préférence de 130°C à 160°C, pour obtenir une phase gazeuse pouvant être mélangée à de l'oxygène, puis portée à plus haute température pour être injectée dans l'oxydeur thermique.

La pompe de chaleur fonctionnant avec un fluide frigorigène pouvant être de l'eau ou tout autre fluide frigorigène est installée sur le deuxième et troisième échangeurs précités. Un flux liquide est vaporisé dans le premier échangeur, puis comprimé dans un compresseur à une pression comprise entre 2 et 30 bars et de préférence 2 à 8 bars et une température de 110°C à 200°C. Le flux obtenu est condensé dans le second échangeur de chaleur puis détendu et refroidi pour redonner le flux liquide, formant ainsi une boucle entre les deux échangeurs.

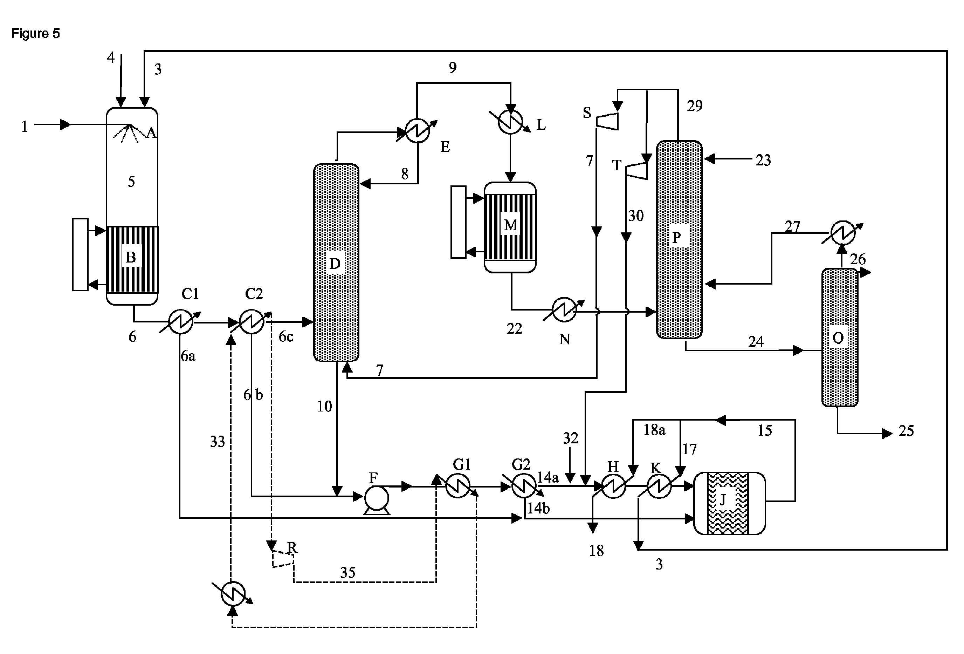

L'utilisation d'une telle pompe à chaleur est décrite plus précisément dans l'exemple 3, en référence à la figure 5 sur laquelle est représentée en pointillés la pompe à chaleur.

Dans une autre configuration représentée sur la figure 3 et dans le cas où la réaction est réalisée en phase gaz, le flux (6) sortant du réacteur de déshydratation est refroidi par un premier échangeur (C) à une température allant de 130°C à 200°C et de préférence 150°C à 180°C, puis injecté directement dans la colonne de condensation (D). L'équilibre thermique de la colonne (D) qui permet de sortir en pied la phase appauvrie en acroléine (10), liquide, et en tête la phase gazeuse enrichie en acroléine (9) est assuré par l'échangeur de refroidissement (E) qui fonctionne à une température allant de 50°C à 100°C et de préférence 60°C à 90°C. Le flux liquide (10) est vaporisé à une pression supérieure de 0,1 à 3 bars à la pression d'entrée du réacteur de déshydratation grâce à l'échangeur de chaleur (G) à une température allant de 120°C à 200°C et de préférence de 130°C à 160°C pour obtenir une phase gazeuse pouvant être mélangée à de l'oxygène, puis portée à plus haute température pour être injectée dans l'oxydeur thermique. La pompe à chaleur pourra être installée sur les échangeurs (E) et (G), l'échangeur (E) permettant de vaporiser le fluide frigorigène qui est ensuite comprimé puis condensé dans l'échangeur (G) avant d'être détendu puis renvoyé dans l'échangeur (E).

Des systèmes de compression peuvent également être utilisés dans une configuration exemplifiée par la figure 3 où la réaction de déshydratation et l'étape d'oxydation sont réalisées en phase gaz et où le flux gazeux (6) sortant du réacteur de déshydratation est refroidi par un (des) échangeur(s) de chaleur (C) puis recomprimé par un compresseur (non représenté sur la figure 3) avant d'être injecté dans la colonne de condensation (D). La colonne (D) et l'(les) échangeur(s) (E) fonctionnent à une pression supérieure d'au moins 1 bar et de préférence au moins 2 bars à celle du réacteur (B). Le flux liquide (10) récupéré en pied est ensuite vaporisé via l'(les) échangeur(s) (G) qui fonctionne(nt) à une pression inférieure à celle des échangeurs (C) et (E) d'au moins 0,5 bar et de préférence d'au moins 1,5 bar et une pression supérieure au réacteur d'oxydation (J) qui fonctionne lui-même à une pression supérieure à celle du réacteur (B). Dans ces conditions, la condensation en tête de la colonne (D) peut être couplée à la vaporisation en sortie de la pompe (F), c'est-à-dire que le flux gazeux sortant en tête de la colonne peut être refroidi directement par le flux liquide sortant de la pompe (F), qui sera lui-même réchauffé. Autrement dit, la vaporisation de la phase aqueuse appauvrie en acroléine issue de l'étape b) est assurée au moins en partie par un échangeur (ou des échangeurs) de chaleur assurant un refroidissement en sortie de l'étape a) et sur l'étape b), c'est à dire que les échangeurs (C), (E) et (G) peuvent être couplés.

Avantageusement, le flux gazeux (6) sortant du réacteur de déshydratation peut être refroidi par l'échangeur de chaleur (C) jusqu'à une température basse, typiquement de 110°C à 160°C, de façon à produire de la vapeur dans l'échangeur (C), vapeur qui pourra être utilisée à d'autres endroits dans le procédé de production de l'acroléine ou l'acide acrylique ou à l'extérieur du procédé.

Le(s) pompe(s) à chaleur mise(s) en oeuvre dans le procédé selon l'invention peu(ven)t également être utilisée(s) pour produire de la vapeur à un niveau thermique suffisant pour qu'elle(s) ai(en)t un usage dans ou à l'extérieur du procédé.

Le procédé selon l'invention contribue ainsi à la diminution de la consommation de combustibles et du rejet de $CO_2$ vers l'atmosphère.

L'acide acrylique bio-ressourcé obtenu selon le procédé de l'invention peut être utilisé pour la fabrication d'homopolymères et copolymères produits par polymérisation de l'acide acrylique et éventuellement d'autres monomères insaturés, par exemple la fabrication de polymères superabsorbants obtenus par polymérisation dudit acide partiellement neutralisé, ou la polymérisation dudit acide suivie d'une neutralisation partielle de l'acide polyacrylique obtenu.

L'acide acrylique bio-ressourcé obtenu selon le procédé de l'invention peut être utilisé aussi pour la fabrication de polymères ou de copolymères par polymérisation des dérivés dudit acide sous forme ester ou amide.

PARTIE EXPERIMENTALE

Une simulation à l'aide du logiciel ASPEN a été utilisée pour illustrer le procédé selon l'invention. Les pourcentages sont exprimes en % massiques. On ne mentionnera pas les espèces dont la teneur est inférieure à 1%. Les pressions sont exprimées en bar absolu.

**EXEMPLE 1 (en référence à la figure 3) : Déshydratation du glycérol en phase gaz du glycérol pour produire de l'acroléine et oxydation thermique en phase gaz sur la phase aqueuse recyclée**

Un flux liquide (1) de glycérol préchauffé à 200°C (17,5 T/h, 98,4% glycérol, 1,1% d'eau) et un flux gazeux (2) (2,1 T/h, 191°C, 7 bars, 85,3% $CO_2$, 9,4% eau, 4,5% $O_2$) sont injectés via une buse de pulvérisation (A) dans un flux gazeux recyclé (3) (70,8 T/h, 491°C, 2,8 bars, 49,5% eau, 47,6% $CO_2$, 2,5% $O_2$), mélangé à un flux d'oxygène (4) (2,6 T/h). La pulvérisation du glycérol en fines gouttelettes permet sa vaporisation sur une courte distance.

Le flux gazeux (5) ainsi obtenu (93,1 T/h, 320°C, 2,7 bars, 18,5% glycérol, 38,1% eau, 4,8% oxygène, 38,1% $CO_2$), est envoyé dans un réacteur multitubulaire à lit fixe (B) contenant 35 m³ d'un catalyseur hétérogène acide de déshydratation et couplé à un bain de sel fondu. De ce réacteur sort un flux gazeux (6) à 320°C sous 1,7 bar (45,3% eau, 3,8% oxygène, 9,0% acroléine, 38,6% $CO_2$). Ce flux est refroidi à 160°C dans un échangeur de chaleur (C) et envoyé dans une colonne d'absorption (D) dans laquelle on injecte en pied un flux gazeux (7) (9,0 T/h, 83°C, 85,3% $CO_2$, 9,4% eau, 4,5% $O_2$) et qui comporte en tête un condenseur (E). De ce condenseur (E) sortent une phase liquide (8) (70°C, 40,1 T/h) qui est renvoyée dans la colonne (D) et un flux gazeux enrichi en acroléine (9) (64,3 T/h) qui sort à 70°C sous 1,6 bar et contient 67,9% de $CO_2$, 13,1% d'acroléine, 9,3% d'eau, 6,2% d'$O_2$, 1,1% d'acétaldéhyde.

Le flux liquide (10) appauvri en acroléine sortant en pied de la colonne (D) (37,8 T/h, 77°C, 97,9% d'eau, 0,005% d'acroléine, 0,5% de glycérol, 0,4% d'acide acétique, 0,3% d'acide acrylique, 0,4% d'acétol, 0,5% d'autres composés organiques lourds) est mélangé à un flux aqueux (11) (1,1 T/h, 96,8% eau, 1,9% méthanol, 1,3% glycérol) puis pompé avec une pompe (F), puis mélangé avec un flux gazeux (12) (57,7 T/h, 190°C, 2,9 bar, 76,2% $CO_2$, 18,8% eau, 1,7% $O_2$, 1,3% CO) et de l'oxygène (13) (4,3 T/h) et porté à 186°C via les échangeurs (G). Le flux (14) obtenu (100,9 T/h, 2,8 bar, 48,4% eau, 43,6% $CO_2$, 5,3% $O_2$, 1,5% organiques, 0,7% CO) est préchauffé à 458°C via l'échangeur (H) et injecté dans un réacteur d'oxydation catalytique adiabatique (J) qui contient 6 $m^3$ d'un catalyseur d'oxydation à base de platine sur alumine. Les fumées (15) sortant du réacteur J (100,9 T/h, 2,8 bar, 700°C, 49,5% eau, 47,6% $CO_2$, 2,5% $O_2$) sont refroidies à 491°C via l'échangeur (H). Le flux obtenu (16) est partagé en un flux (3) déjà décrit et en un flux (17) (30,1 T/h, 2.8 bars, 491°C). Le flux (17) est refroidi à 83°C au moyen des échangeurs (K). La phase liquide (18) constituée à 99,9% d'eau est éliminée. La phase gaz (19) (83°C, 2,7 bar, 85,3% $CO_2$, 9,4% eau, 4,5% $O_2$) est partagée en un flux (7) déjà décrit, un flux (20) qui est éliminé et un flux (21) qui est comprimé pour former le flux (2) déjà décrit.

## EXEMPLE 2 (en référence à la figure 4) : Production d'acide acrylique

Le flux gazeux (9) de l'exemple 1 (64,3 T/h, 70°C, 1,6 bar, 67,9% $CO_2$, 13,1% acroléine, 9,3% eau, 6,2% $O_2$, 1,1% acétaldéhyde) est réchauffé à 160°C par un échangeur (L), puis injecté dans un deuxième réacteur multitubulaire à lit fixe (M) comportant un catalyseur d'oxydation et couplé à un bain de sel fondu permettant d'évacuer la chaleur produite par la réaction. En sortie de ce réacteur, le flux gazeux (22) (64,3 T/h, 68,4% $CO_2$, 15,8% acide acrylique, 9,5 % eau, 1,6% oxygène, 1,1% monoxyde de carbone, 1,0% acide acétique) est refroidi à 160°C par l'échangeur (N), puis injecté en pied de la colonne d'absorption (P). En tête de cette colonne, on injecte un flux (23) de 9 T/h d'eau à 25°C. On récupère en pied une phase liquide (24) (16.6 T/h, 80°C, 62.0% acide acrylique, 30,9% eau, 4,0% acide acétique, 2,4% acide formique). Cette phase liquide est envoyée vers une colonne (Q) fonctionnant sous vide qui permet de récupérer un flux d'acide acrylique (25) (15,6 T/h, 65% acide acrylique, 27,8% eau, 4,2% acide acétique, 2,6% acide formique). En tête de la colonne (Q), le flux gazeux (26) (0,9 T/h, 69°C, 0,3 bars) est envoyé dans un condenseur qui permet d'obtenir une phase liquide (27) qui est renvoyée dans la colonne (P) et une phase gaz (28) qui alimente un groupe de vide (R). L'évent de ce groupe de vide est combiné avec la phase gaz (29) de la colonne (P) (57,7 T/h, 74°C, 76,2% $CO_2$, 18,8% eau, 1,7% $O_2$, 1,3% CO). Ce flux est recomprimé par le compresseur (S) et forme le flux (12) décrit à l'exemple 1.

## EXEMPLE 3 (en référence à la figure 5) : Déshydratation du glycérol en phase gaz pour produire de l'acroléine qui est oxydée en acide acrylique et oxydation thermique en phase gaz sur la phase aqueuse recyclée et avec pompe à chaleur

Un flux liquide (1) de glycérol préchauffé à 210°C (17,4 T/h, 99,0% glycérol) est injecté via une buse de pulvérisation (A) dans un flux gazeux recyclé (3) (62,5 T/h, 485°C, 2,8 bars, 68,1% eau, 28,7% $CO_2$, 2,9% $O_2$), mélangé à un flux d'oxygène (4) (2,6 T/h). La pulvérisation du glycérol en fines gouttelettes permet sa vaporisation sur une courte distance.

Le flux gazeux (5) ainsi obtenu (82,5 T/h, 320°C, 2,8 bars, 20,8% glycérol, 51,7% eau, 5,3% oxygène, 21,8% $CO_2$), est envoyé dans un réacteur multitubulaire à lit fixe (B) contenant un catalyseur hétérogène de déshydratation et couplé à un bain de sel fondu. De ce réacteur sort un flux gazeux (6) à 320°C sous 1,8 bar (59,8% eau, 4,2% oxygène, 10,8% acroléine, 22,4% $CO_2$). Ce flux est refroidi à 160°C dans un échangeur de chaleur (C1), duquel on récupère un petit flux de produits lourds liquides (6a) (68 kg/h) et une phase gaz, qui est refroidie à 102°C via un échangeur de chaleur (C2). On obtient une phase liquide (6b) (26,7 T/h, 97% d'eau) et une phase gaz (6c) (42,1 % eau, 33,1% $CO_2$, 6,3% $O_2$, 14,7% acroléine, 1,3% acétaldéhyde, 1,1% CO) qui est envoyée dans une colonne d'absorption (D). On injecte également dans cette colonne (D) un flux gazeux (7) (35,9 T/h, 123°C, 76,3% $CO_2$, 16,5% eau, 3,0% CO, 1,9% $O_2$). En tête de (D), un condenseur (E) génère une phase liquide (8) (74°C, 1,7 bar, 22,6 T/h) qui est renvoyée dans la colonne (D) et un flux gazeux enrichi en acroléine (9) (69,5 T/h, 74°C, 1,7 bar) qui contient 65,9% de $CO_2$, 12,1% d'acroléine, 10,8% d'eau, 6,0% d'$O_2$, 2,4% CO, 1,3% d'acétaldéhyde.

Le flux gazeux (9) est réchauffé à 240°C par un échangeur (L), puis injecté dans un deuxième réacteur multitubulaire à lit fixe (M) comportant un catalyseur d'oxydation et couplé à un bain de sel fondu permettant d'évacuer la chaleur produite par la réaction. En sortie de ce réacteur, le flux gazeux (22) (69,5 T/h, 66,4% $CO_2$, 14,6% acide acrylique, 11,0 % eau, 2,6% monoxyde de carbone, 1,7% oxygène, 1,1% acide acétique) est refroidi à 160°C par l'échangeur (N), puis injecté dans la colonne d'absorption (P). En tête de cette colonne, on injecte un flux (23) de 6,5 T/h d'eau à 25°C. On récupère en pied une phase liquide (24) qui est envoyée vers une colonne (Q) fonctionnant sous vide qui permet de récupérer un flux d'acide acrylique (25) (15,5 T/h, 64,9% acide acrylique, 26,7% eau, 4,9% acide acétique, 3,0% acide formique). En tête de la colonne (Q), le flux gazeux (26) (1,6 T/h, 72°C, 0,3 bars) est envoyé dans un condenseur puis dans la colonne (P). La phase gaz (29) de la colonne (P) (60,5 T/h, 72°C, 1,1 bar, 76,3% $CO_2$, 16,5% eau, 1,9% $O_2$, 3,0% CO) est en partie recomprimée à 1,7 bar par le compresseur (S) et forme le flux (7) décrit plus haut. L'autre partie est comprimée à 2,9 bar via le compresseur (T) pour donner le flux (30)

Le flux liquide (10) appauvri en acroléine sortant en pied de la colonne (D) (22,2 T/h, 85°C, 98,9% d'eau, 0,03% d'acroléine) est mélangé au flux (6b) pompé avec une pompe (F), puis vaporisé dans Les échangeurs (G1) et (G2) pour donner un flux (14a) gazeux (48,3 T/h, 135°C, 2,9 bar, 98,2% eau) et un flux (14b) liquide qui est mélangé au flux (6a) et directement injecté dans l'oxydeur thermique (J). Les flux (14a) et (30) sont mélangés avec un flux (32) d'oxygène (4,6 T/h) pour former un flux (14c) qui est chauffé via les échangeurs (H) et (K) à 977°C puis injectés dans l'oxydeur thermique. En sortie de l'oxydeur thermique, le flux (15) obtenu (79,3 T/h, 1199°C, 68,1 % eau, 28,7% $CO_2$, 2,9% $O_2$) est partagé en un flux (18a) qui est refroidi via l'échangeur(H) à 189°C (16,8 T/h) puis éliminé, et un flux (17) qui est refroidi via l'échangeur (K) à 485°C pour former le flux (3) déjà décrit.

Une pompe à chaleur fonctionnant à la vapeur d'eau est installée sur les échangeurs (C2) et (G1) et est représentée en pointillés. Un flux (33) d'eau liquide (26,2 T/h, 25°C) est vaporisé l'échangeur (C2) (100°C, 1 bar) puis comprimé par le compresseur (R) à 3,5 bars, 270°C. Le flux (35) obtenu est condensé dans l'échangeur (G1) puis détendu et refroidi pour redonner le flux (33).

**EXEMPLE 4** (selon l'invention) :

Un catalyseur acide de déshydratation est préparé par imprégnation au volume poreux d'une solution aqueuse d'acide phosphotungstique (3,9 g d'acide phosphotungstique dans 5,7 g d'eau) sur un oxyde de titane (15,4 g) réduit à la granulométrie de 300-500 $\mu$m. Le catalyseur est séché à l'étuve ventilée à 110°C puis calciné pendant 3 heures à 500°C. Un volume de 7 ml du catalyseur de déshydratation est introduit dans un réacteur en inox 316L de diamètre 13 mm placé verticalement dans un four chauffé à 280°C.

Un débit de 15 g/h d'une solution constituée de 50% massique de glycérol pur et 50% d'eau est mélangé avec un débit d'oxygène et d'azote de respectivement 1,2 et 18 normaux litres / heure, puis envoyé dans un vaporiseur qui chauffe le mélange à 280°C et qui est connecté au réacteur.

Les effluents gazeux en sortie du réacteur sont soit envoyés vers deux pièges en série contenant initialement 120 et 80 grammes d'eau et refroidis à 0°C de façon à piéger totalement l'acroléine pour faire un bilan matière, soit envoyé vers un réservoir refroidi à 0°C de façon à piéger la majeure partie de l'eau et des lourds produits par la réaction. On mesure la perte de charge du réacteur au long de l'expérience.

On a effectué un bilan matière avec les pièges en série remplis d'eau du temps t = 2 heures à t = 3 heures 30 d'une part et du temps t = 21 heures à t = 22 heures 30. On a mesuré par chromatographie gaz la teneur en glycérol et en acroléine dans les pièges. On calcule la conversion en glycérol et le rendement en acroléine selon les formules :

$$\text{Conversion glycérol (\%)} = \left(\text{(moles de glycérol injectées dans le réacteur pendant la durée du bilan)} - \text{(moles de glycérol récupérées dans les 2 pièges)}\right) / \text{(moles de glycérol injectées dans le réacteur pendant la durée du bilan)} * 100$$

$$\text{Rendement acroléine (\%)} = \text{(moles d'acroléine récupérée dans les 2 pièges)} / \text{(moles de glycérol injectées dans le réacteur pendant la durée du bilan)} * 100$$

Les résultats de l'expérience sont reportés au tableau 1 ci-dessous.

Par ailleurs on a collecté dans le réservoir la majeure partie de l'eau et des lourds entre le temps t = 1 heure et le temps t = 2 heures, ainsi qu'entre le temps t = 3 heures 30 et le temps t = 21 heures. Cette solution aqueuse a ensuite été traitée pendant 2 heures à l'évaporateur rotatif chauffé à 30°C et sous vide partiel, de façon à évaporer l'acroléine. La collecte dans le réservoir et l'évaporation permettent de simuler l'étape (D) représentée à la figure 1.

Afin de simuler les étapes (J), (A) et (B) de la figure 1, on a couplé au réacteur contenant le catalyseur de déshydratation un deuxième réacteur contenant 1,5 ml d'un catalyseur d'oxydation CK307 d'Haldor Topsoe. Un flux de 5,5 g/h de la solution aqueuse récupérée est mélangé avec un débit d'oxygène et d'azote de respectivement 2,4 et 18 normaux litres / heure, puis envoyé dans un vaporiseur qui chauffe le mélange à 300°C et qui est connecté au réacteur contenant le catalyseur d'oxydation. Un flux de 9,4g/h d'une solution aqueuse à 80% de glycérol est injecté entre le réacteur d'oxydation et le réacteur de déshydratation. Le réacteur d'oxydation est maintenu dans une zone à 300°C et celui de déshydratation dans une zone à 280°C.

Les bilans matières sont réalisés comme précédemment et les résultats sont reportés au tableau 1.

**EXEMPLE 5** (comparatif) :

De la même façon qu'à l'exemple 4, on a réalisé la réaction de déshydratation du glycérol avec une solution constituée de 50% massique de glycérol pur et 50% d'eau et on a collecté dans un réservoir la majeure partie de l'eau et des lourds, qui ont été pendant 2 heures à l'évaporateur rotatif chauffé à 30 °C et sous vide partiel, de façon à évaporer l'acroléine.

On a alors mélangé directement la solution aqueuse récupérée contenant un mélange eau et lourds recyclés avec du glycérol pur pour préparer une solution à 50% de glycérol et on a renouvelé l'expérience de déshydratation du glycérol. Pendant 3 heures 30, on a observé une perte de charge de 0,1 bar sur le réacteur, puis la pression a augmenté progressivement pour atteindre 0,3 bar après 5 heures et dépasser 1 bar après 7 heures. L'expérience n'a pas pu être poursuivie à cause de l'augmentation exponentielle de la perte de charge.

Les résultats sont reportés au tableau 1.

Lorsqu'on utilise de l'eau recyclée contenant des lourds, on a observé une montée très rapide en pression consécutive à un bouchage du réacteur.

**Tableau 1**

| | Conversion glycérol après 2h / 21 h (%) | Rendement acroléine après 2h / 21 h (%) | Perte de charge après 2h/ 5h/ 21h (bar) |
|---|---|---|---|
| Exemple 4 Glycérol / eau | >99% / 80% | 70% / 48% | 0,1 / 0,1 / 0,1 |
| Exemple 4 Glycérol / condensat eau recyclée avec réacteur d'oxydation | >99% / 79% | 70% / 46% | 0,1 / 0,1 / 0,1 |
| Exemple 5 (comparatif) Glycérol / eau et lourds recyclés sans étape d'oxydation | >99% / - | 70% / - | 0,1 / 0,3 / >1 |

**EXEMPLE 6: Déshydratation du glycérol en phase gaz pour produire de l'acroléine qui est oxydée en acide acrylique et oxydation thermique en phase gaz à pression atmosphérique sur la phase aqueuse recyclée**

Un flux liquide (1) de glycérol préchauffé à 275°C (17,4 T/h, 99,0% glycérol) est injecté via un mélangeur venturi dans un flux gazeux recyclé (3) (50,6 T/h, 442°C, 3,0 bars, 59,1% azote, 25,3% eau, 12,1% $CO_2$, 2,5% $O_2$, 1,0% argon), mélangé à un flux d'air (4) (20,6 T/h, 172°C, 3,0 bars) et de vapeur (4,3 T/h, 134°C, 3,0 bar). Le mélangeur à effet Venturi permet la vaporisation du glycérol sur une courte distance. Le flux gazeux (5) ainsi obtenu (92,8 T/h, 240°C, 2,8 bars, 18,5% glycérol, 18,5% eau, 6,4% oxygène, 6,6% $CO_2$, 49,0% azote), est envoyé dans un réacteur multitubulaire à lit fixe (B) contenant un catalyseur hétérogène de déshydratation et couplé à un bain de sel fondu. De ce réacteur sort un flux gazeux (6) à 320°C sous 1,8 bar (49,0% azote, 25,7% eau, 5,4% oxygène, 9,0% acroléine, 7,2% $CO_2$). Ce flux est refroidi à 193°C dans un échangeur de chaleur (C1), puis à 120°C dans un échangeur de chaleur (C2) duquel on récupère un petit flux de produits lourds liquides (6b) (0,2 T/h) et une phase gaz (6c) (92,6 T/h, 120°C, 1,7 bars, 49,1% azote, 25,8% eau, 7,2% $CO_2$, 5,4% $O_2$, 9,1 % acroléine) qui est envoyée dans une colonne d'absorption (D). En tête de (D), un condenseur (E) génère une phase liquide (8) (74°C, 1,7 bar, 14,3 T/h) qui est renvoyée dans la colonne (D) et un flux gazeux enrichi en acroléine (9) (79,4 T/h, 74°C, 1,7 bar) qui contient 8,4% de $CO_2$, 10,5% d'acroléine, 14,2% d'eau, 6,3% d'$O_2$, 57,3% d'azote. Le flux gazeux (9) est réchauffé à 240°C par l'échangeur de chaleur (C1) déjà décrit, puis injecté dans un deuxième réacteur multitubulaire à lit fixe (M) comportant un catalyseur d'oxydation et couplé à un bain de sel fondu permettant d'évacuer la chaleur produite par la réaction. En sortie de ce réacteur, le flux gazeux (22) (79,4 T/h, 8,8% $CO_2$, 12,7% acide acrylique, 14,4% eau, 57,3% azote, 2,6% oxygène) est refroidi à 160°C par l'échangeur (N), puis injecté dans la colonne d'absorption (P). En tête de cette colonne, on injecte un flux (23) de 10,4 T/h d'eau à 25°C. On récupère en pied une phase liquide (24) qui est envoyée vers une colonne (Q) fonctionnant sous vide qui permet de récupérer en pied un flux d'acide acrylique (25) (18,2 T/h, 55,1% acide acrylique, 38,6% eau, 3,7% acide acétique, 2,2% acide formique). En tête de la colonne (Q), le flux gazeux (26) est envoyé dans un condenseur puis dans la colonne (P). La phase gaz (29) de la colonne (P) (71,6 T/h, 70°C, 1,1 bar) est composée de 9,8% $CO_2$, 20,6% eau, 2,9% $O_2$, 1,0% argon, 1,0% CO, 63,5% azote.

Le flux liquide (10) appauvri en acroléine sortant en pied de la colonne (D) (13,2 T/h, 89°C, 95,4% d'eau, 1,0% d'hydroxypropanone) est mélangé au flux (6b) et à un flux issu de la purification du flux d'acide acrylique (25) (1,2 T/h, 100% de composés organiques) et à un autre flux d'eau recyclé (1,2 T/h). Le mélange est vaporisé via l'échangeur (G) pour donner un flux (14a) gazeux (15,4 T/h, 133°C, 1,9 bar, 88,4% eau, 1,3% acide acrylique, 5,1% acide acétique, 2,7% acide formique) et un flux (14b) liquide (0,4 T/h) qui est directement injecté dans l'oxydeur thermique (J). Les flux (14a) et (29) sont mélangés avec un flux (32) d'air (40,5 T/h, 1,2 bar) et de gaz naturel (1,0 T/h) pour former un flux (14c) qui est chauffé via l'échangeur (H) à 450°C puis injecté dans l'oxydeur thermique qui fonctionne à pression atmosphérique. En sortie de l'oxydeur thermique, le flux (15) obtenu (128,9 T/h, pression atmosphérique, 1026°C, 25,3% eau, 12,1% $CO_2$, 2,5% $O_2$, 59,1% azote, 1,0% argon) est refroidi à 702°C par l'échangeur (H) déjà décrit et par l'échangeur (H1) à 670°C puis est partagé en un flux (18a) (78,4 T/h) qui est refroidi à 170°C via les échangeurs (H2) et (H3) puis éliminé via une cheminée, et un flux (17) (50,6 T/h). Le flux (17) est refroidi via l'échangeur (K) à 160°C puis comprimé à 3,0 bars et 352°C et surchauffé à 442°C via l'échangeur (H1) déjà décrit pour former le flux (3) déjà décrit.

**Revendications**

**1.** Procédé de fabrication d'acroléine à partir de glycérol comprenant au moins les étapes suivantes :

a) on soumet du glycérol à une réaction de déshydratation pour obtenir un flux aqueux contenant de l'acroléine,
b) on sépare le flux issu de l'étape a) en une phase riche en acroléine et une phase aqueuse appauvrie en acroléine,
c) on recycle tout ou partie de la phase aqueuse appauvrie en acroléine à l'étape a), **caractérisé en ce que** l'on met en oeuvre une étape d'oxydation en présence d'oxygène, d'un gaz contenant de l'oxygène, de peroxyde d'hydrogène ou d'ozone, sur ladite phase aqueuse appauvrie en acroléine avant d'être recyclée à l'étape a).

**2.** Procédé selon la revendication 1 comprenant en outre un traitement de purification de ladite phase riche en acroléine par absorption/distillation.

**3.** Procédé de fabrication d'acide acrylique à partir de glycérol comprenant au moins les étapes suivantes :

a) on soumet du glycérol à une réaction de déshydratation pour obtenir un flux aqueux contenant de l'acroléine,
b) on sépare le flux issu de l'étape a) en une phase riche en acroléine et une phase aqueuse appauvrie en acroléine,
c) on recycle tout ou partie de la phase aqueuse appauvrie en acroléine à l'étape a),
d) on soumet la phase riche en acroléine à une réaction d'oxydation catalytique pour obtenir un flux contenant de l'acide acrylique,
e) on soumet le flux issu de l'étape d) à un ou plusieurs traitements de purification, et l'on récupère l'acide acrylique purifié,

**caractérisé en ce que** l'on met en oeuvre une étape d'oxydation en présence d'oxygène, d'un gaz contenant de l'oxygène, de peroxyde d'hydrogène ou d'ozone, sur ladite phase aqueuse appauvrie en acroléine avant d'être recyclée à l'étape a).

**4.** Procédé selon l'une des revendications 1 à 3 **caractérisé en ce que** l'étape d'oxydation sur la phase aqueuse appauvrie en acroléine est réalisée selon une oxydation thermique en phase gaz en présence d'oxygène à une température supérieure à 700°C.

**5.** Procédé selon l'une des revendications 1 à 3 **caractérisé en ce que** l'étape d'oxydation sur la phase aqueuse appauvrie en acroléine est réalisée selon une oxydation catalytique en phase gaz en présence d'oxygène à une température allant de 200°C à 500°C en présence d'un catalyseur d'oxydation.

**6.** Procédé selon l'une des revendications 1 à 3 **caractérisé en ce que** l'étape d'oxydation sur la phase aqueuse appauvrie en acroléine est réalisée selon une oxydation en voie humide ou supercritique à une température supérieure à 150°C et une pression supérieure à 5 bars, en présence d'oxygène ou d'air.

**7.** Procédé selon l'une des revendications 1 à 3 **caractérisé en ce que** l'étape d'oxydation sur la phase aqueuse appauvrie en acroléine est réalisée selon une oxydation en phase liquide en présence de peroxyde d'hydrogène ou d'ozone, ou la combinaison de ces deux réactifs, éventuellement activés par l'utilisation de rayonnement UV ou de catalyseurs tels les sels de fer II.

**8.** Procédé selon l'une quelconque des revendications précédentes **caractérisé en ce que** l'énergie contenue dans le flux de sortie de l'étape d'oxydation de la phase aqueuse appauvrie en acroléine est utilisée pour préchauffer le flux d'entrée de cette étape.

**9.** Procédé selon l'une quelconque des revendications précédentes **caractérisé en ce que** la phase aqueuse recyclée à l'étape de déshydratation a) sous forme de flux gazeux (3) est utilisée pour vaporiser le flux de glycérol dans une étape de mélange (A) avant d'envoyer le flux gazeux réactif dans le réacteur de déshydratation (B).

**10.** Procédé selon la revendication 9 **caractérisé en ce que** le flux de glycérol est injecté dans la chambre de mélange (A) via des buses de pulvérisation ou d'atomisation comprenant éventuellement, dans le cas des buses d'atomisation, l'injection d'un flux gazeux contenant majoritairement du $CO_2$ issu de l'étape d'oxydation de la phase aqueuse appauvrie en acroléine.

**11.** Procédé selon l'une quelconque des revendications précédentes **caractérisé en ce que** l'étape a) est réalisée en phase gaz et on utilise une pompe à chaleur permettant de condenser le flux réactionnel gazeux à l'issue de l'étape a) et de vaporiser la phase aqueuse appauvrie en acroléine séparée de la phase enrichie en acroléine lors de l'étape b).

**12.** Procédé selon l'une quelconque des revendications 1 à 5 et 8 à 11 **caractérisé en ce que** l'étape a) et l'étape d'oxydation sur la phase aqueuse appauvrie en acroléine sont réalisées en phase gaz, et on utilise une pompe à chaleur permettant de condenser la phase appauvrie en acroléine lors de l'étape b) et de vaporiser ladite phase aqueuse appauvrie en acroléine à la sortie de l'étape b).

**13.** Procédé selon l'une quelconque des revendications 1 à 5 et 8 à 11 **caractérisé en ce que** l'étape a) et l'étape d'oxydation sur la phase aqueuse appauvrie en acroléine sont réalisées en phase gaz, et la vaporisation de la phase aqueuse appauvrie en acroléine issue de l'étape b) est assurée au moins en partie par un échangeur (ou des échangeurs) de chaleur assurant un refroidissement en sortie de l'étape a) et sur l'étape b).

**14.** Procédé selon la revendication 13 **caractérisé en ce que** le refroidissement des flux en sortie de l'étape a) et sur l'étape b) est opéré à une pression supérieure d'au moins 0,5 bar par rapport à celle pour la vaporisation de la phase aqueuse appauvrie en acroléine issue de l'étape b).

## Patentansprüche

**1.** Verfahren zur Herstellung von Acrolein aus Glycerin, das mindestens die folgenden Schritte umfasst:

a) man unterwirft das Glycerin einer Dehydratisierungsreaktion, wobei man einen Acrolein enthaltenden wässrigen Strom erhält,
b) man trennt den Strom aus Schritt a) in eine acroleinreiche Phase und eine an Acrolein abgereicherte wässrige Phase,
c) man führt die gesamte an Acrolein abgereicherte wässrige Phase oder einen Teil davon in Schritt a) zurück,

**dadurch gekennzeichnet, dass** man an der an Acrolein abgereicherten wässrigen Phase vor der Rückführung in Schritt a) einen Oxidationsschritt in Gegenwart von Sauerstoff, einem sauerstoffhaltigen Gas, Wasserstoffperoxid oder Ozon durchführt.

**2.** Verfahren nach Anspruch 1, außerdem umfassend eine Reinigungsbehandlung der acroleinreichen Phase durch Absorption/Destillation.

**3.** Verfahren zur Herstellung von Acrylsäure aus Glycerin, das mindestens die folgenden Schritte umfasst:

a) man unterwirft das Glycerin einer Dehydratisierungsreaktion, wobei man einen Acrolein enthaltenden wässrigen Strom erhält,
b) man trennt den Strom aus Schritt a) in eine acroleinreiche Phase und eine an Acrolein abgereicherte wässrige Phase,
c) man führt die gesamte an Acrolein abgereicherte wässrige Phase oder einen Teil davon in Schritt a) zurück,
d) man unterwirft die acroleinreiche Phase einer katalytischen Oxidationsreaktion, wobei man einen Acrylsäure enthaltenden Strom erhält,
e) man unterwirft den Strom aus Schritt d) einer oder mehreren Reinigungsbehandlungen und gewinnt die gereinigte Acrylsäure,

**dadurch gekennzeichnet, dass** man an der an Acrolein abgereicherten wässrigen Phase vor der Rückführung in Schritt a) einen Oxidationsschritt in Gegenwart von Sauerstoff, einem sauerstoffhaltigen Gas, Wasserstoffperoxid oder Ozon durchführt.

**4.** Verfahren nach einem der Ansprüche 1 bis 3, **dadurch gekennzeichnet, dass** der Oxidationsschritt an der an Acrolein abgereicherten wässrigen Phase gemäß einer thermischen Oxidation in der Gasphase in Gegenwart von Sauerstoff bei einer Temperatur von mehr als 700°C durchgeführt wird.

**5.** Verfahren nach einem der Ansprüche 1 bis 3, **dadurch gekennzeichnet, dass** der Oxidationsschritt an der an Acrolein abgereicherten wässrigen Phase gemäß einer katalytischen Oxidation in der Gasphase in Gegenwart von Sauerstoff bei einer Temperatur im Bereich von 200°C bis 500°C in Gegenwart eines Oxidationskatalysators durchgeführt wird.

**6.** Verfahren nach einem der Ansprüche 1 bis 3, **dadurch gekennzeichnet, dass** der Oxidationsschritt an der an Acrolein abgereicherten wässrigen Phase gemäß einer Oxidation auf nassem oder überkritischem Wege bei einer Temperatur von mehr als 150°C und einem Druck von mehr als 5 bar in Gegenwart von Sauerstoff oder Luft durchgeführt wird.

**7.** Verfahren nach einem der Ansprüche 1 bis 3, **dadurch gekennzeichnet, dass** der Oxidationsschritt an der an Acrolein abgereicherten wässrigen Phase gemäß einer Oxidation in der Flüssigphase in Gegenwart von Wasserstoffperoxid oder Ozon oder einer Kombination dieser beiden Reaktanden, die gegebenenfalls durch Verwendung von UV-Strahlung oder Katalysatoren wie Eisen-II-Salzen aktiviert werden, durchgeführt wird.

**8.** Verfahren nach einem der vorhergehenden Ansprüche, **dadurch gekennzeichnet, dass** die in dem Ausgangsstrom des Schritts der Oxidation der an Acrolein abgereicherten wässrigen Phase enthaltene Energie zum Vorwärmen des Eingangsstroms dieses Schritts verwendet wird.

**9.** Verfahren nach einem der vorhergehenden Ansprüche, **dadurch gekennzeichnet, dass** die in Form des Gasstroms (3) in den Dehydratisierungsschritt a) zurückgeführte wässrige Phase zum Verdampfen des Glycerinstroms in einem Mischschritt (A) vor dem Einleiten des reaktiven Gasstroms in den Dehydratisierungsreaktor (B) verwendet wird.

**10.** Verfahren nach Anspruch 9, **dadurch gekennzeichnet, dass** der Glycerinstrom über Sprüh- oder Zerstäubungsdüsen in die Mischkammer (A) eingeleitet wird, wobei im Fall von Zerstäubungsdüsen gegebenenfalls ein hauptsächlich $CO_2$ enthaltender Gasstrom aus dem Schritt der Oxidation der an Acrolein abgereicherten wässrigen Phase eingeleitet wird.

**11.** Verfahren nach einem der vorhergehenden Ansprüche, **dadurch gekennzeichnet, dass** der Schritt a) in der Gasphase durchgeführt wird und man eine Wärmepumpe verwendet, mit der der Reaktionsgasstrom aus Schritt a) kondensiert und die von der acroleinreichen Phase abgetrennte, an Acrolein abgereicherte wässrige Phase während Schritt b) verdampft werden kann.

**12.** Verfahren nach einem der Ansprüche 1 bis 5 und 8 bis 11, **dadurch gekennzeichnet, dass** der Schritt a) und der Oxidationsschritt an der an Acrolein abgereicherten wässrigen Phase in der Gasphase durchgeführt werden und man eine Wärmepumpe verwendet, mit der die an Acrolein abgereicherte wässrige Phase während Schritt b) kondensiert und die an Acrolein abgereicherte wässrige Phase am Ausgang von Schritt b) verdampft werden kann.

**13.** Verfahren nach einem der Ansprüche 1 bis 5 und 8 bis 11, **dadurch gekennzeichnet, dass** der Schritt a) und der Oxidationsschritt an der an Acrolein abgereicherten wässrigen Phase in der Gasphase durchgeführt werden und die Verdampfung der an Acrolein abgereicherte wässrigen Phase am Ausgang von Schritt b) zumindest teilweise durch einen Wärmetauscher (oder mehrere Wärmetauscher), die am Ausgang von Schritt a) und in Schritt b) für eine Abkühlung sorgen, gewährleistet wird.

**14.** Verfahren nach Anspruch 13, **dadurch gekennzeichnet, dass** die Abkühlung der Ströme am Ausgang von Schritt a) und in Schritt b) bei einem Druck betrieben wird, der mindestens 0,5 bar über dem Druck für die Verdampfung der an Acrolein abgereicherten wässrigen Phase aus Schritt b) liegt.

**Claims**

**1.** Process for the manufacture of acrolein from glycerol comprising at least the following stages:

a) glycerol is subjected to a dehydration reaction in order to obtain an aqueous stream comprising acrolein;

b) the stream resulting from stage a) is separated into an acrolein-rich phase and an acrolein-depleted aqueous phase; and
c) all or part of the acrolein-depleted aqueous phase is recycled to stage a), **characterized in that** an oxidation stage is carried out, in the presence of oxygen, an oxygen-containing gas, hydrogen peroxide or ozone, on said acrolein-depleted aqueous phase before being recycled to stage a).

2. Process according to Claim 1, additionally comprising a purification treatment of said acrolein-rich phase by absorption/distillation.

3. Process for the manufacture of acrylic acid from glycerol comprising at least the following stages:

a) glycerol is subjected to a dehydration reaction in order to obtain an aqueous stream comprising acrolein;
b) the stream resulting from stage a) is separated into an acrolein-rich phase and an acrolein-depleted aqueous phase;
c) all or part of the acrolein-depleted aqueous phase is recycled to stage a);
d) the acrolein-rich phase is subjected to a catalytic oxidation reaction in order to obtain a stream comprising acrylic acid;
e) the stream resulting from stage d) is subjected to one or more purification treatments and purified acrylic acid is recovered,

**characterized in that** an oxidation stage is carried out, in the presence of oxygen, an oxygen-containing gas, hydrogen peroxide or ozone, on said acrolein-depleted aqueous phase before being recycled to stage a).

4. Process according to one of Claims 1 to 3, **characterized in that** the oxidation stage carried out on the acrolein-depleted aqueous phase is a thermal oxidation in the gas phase in the presence of oxygen at a temperature above 700°C.

5. Process according to one of Claims 1 to 3, **characterized in that** the oxidation stage carried out on the acrolein-depleted aqueous phase is a catalytic oxidation in the gas phase in the presence of oxygen at a temperature ranging from 200°C to 500°C in the presence of an oxidation catalyst.

6. Process according to one of Claims 1 to 3, **characterized in that** the oxidation stage carried out on the acrolein-depleted aqueous phase is a wet oxidation or supercritical oxidation at a temperature above 150°C and a pressure above 5 bar, in the presence of oxygen or air.

7. Process according to one of Claims 1 to 3, **characterized in that** the oxidation stage carried out on the acrolein-depleted aqueous phase is an oxidation in the liquid phase in the presence of hydrogen peroxide or ozone, or a combination of these two reactants, optionally activated by the use of UV radiation or of catalysts such as iron(II) salts.

8. Process according to any one of the preceding claims, **characterized in that** the energy contained in the stream leaving the stage for oxidizing the acrolein-depleted aqueous phase is used to preheat the stream entering this stage.

9. Process according to any one of the preceding claims, **characterized in that** the aqueous phase recycled to the dehydration stage a) in the form of the gas stream (3) is used to vaporize the glycerol stream in a mixing stage (A) before conveying the reactive gas stream to the dehydration reactor (B).

10. Process according to Claim 9, **characterized in that** the glycerol stream is injected into the mixing chamber (A) via spray or atomization nozzles optionally including, in the case of atomization nozzles, the injection of a gas stream containing predominantly $CO_2$ coming from the stage for oxidizing the acrolein-depleted aqueous phase.

11. Process according to any one of the preceding claims, **characterized in that** stage a) is carried out in the gas phase and a heat pump is used to condense the gaseous reaction stream coming from stage a) and to vaporize the acrolein-depleted aqueous phase separated from the acrolein-enriched phase during stage b).

12. Process according to any one of Claims 1 to 5 and 8 to 11, **characterized in that** stage a) and the stage for oxidizing the acrolein-depleted aqueous phase are carried out in the gas phase, and a heat pump is used to condense the acrolein-depleted phase during stage b) and to vaporize said acrolein-depleted aqueous phase on leaving stage b).

**13.** Process according to any one of Claims 1 to 5 and 8 to 11, **characterized in that** stage a) and the stage for oxidizing the acrolein-depleted aqueous phase are carried out in the gas phase, and the acrolein-depleted aqueous phase coming from stage b) is vaporized at least partly by a heat exchanger (or exchangers), providing cooling on leaving stage a) and in stage b).

**14.** Process according to Claim 13, **characterized in that** the cooling of the streams on leaving stage a) and in stage b) is operated at a pressure at least 0.5 bar above that for the vaporization of the acrolein-depleted aqueous phase coming from stage b).

Figure 1

Figure 2

Figure 3

**Figure 4**

EP 2 513 027 B1

Figure 5

EP 2 513 027 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006092271 A
- FR 695931
- US 2558520 A
- WO 9905085 A
- US 5387720 A
- WO 06087083 A
- WO 06087084 A
- WO 09044081 A
- EP 1710227 A
- WO 06136336 A
- WO 2006092272 A
- WO 08087315 A
- US 6348638 B
- EP 1978009 A

- WO 06114506 A
- US 2008018319 A
- WO 2006136336 A
- US 3433840 A
- EP 1300384 A
- EP 1474374 A
- EP 995491 A
- EP 1147807 A
- US 20050020851 A
- FR 2146386
- US 5426221 A
- FR 9614397
- FR 2928648

**Littérature non-brevet citée dans la description**

- **K. TANABE et al.** Studies in Surface Science and Catalysis. 1969, vol. 51